# EUROPEAN PATENT APPLICATION

(11) **EP 4 737 553 A2**
(43) Date of publication of application: **06.05.2026**
(21) Application number: 26152461.5
(22) Date of filing: 22.03.2024
(51) Int. Cl.: C12M 1/34

(54) **SYSTEM AND METHOD FOR BIOPSY PROCESSING**

(30) Priority: 23.03.2023 US 202363491838 P
(62) Divisional of application: 24717998.9
(71) Applicant: DEKA Products Limited Partnership, Manchester, NH 03101 (US)
(72) Inventor: WHITE, Matthew, Henniker, 03242 (US); KIM, Kevin, Hooksett, 03106 (US); FINSETH, Bryan A, Newbury, 03255 (US)
(74) Representative: EIP

(57) **Abstract**

A system for digesting a biopsy sample. The system comprises: a vial configured to receive the biopsy sample and a transport fluid, a cap coupled to the vial, the cap comprising a movable tube having an aspiration tip at a distal end, a tube gripper configured to engage a neck of the movable tube and move the movable tube up and down within the vial, an input syringe pump configured to pump air into the vial through the movable tube and to supply a wash fluid and a digest solution to the vial through the movable tube, an output syringe configured to withdraw the transport fluid, the wash fluid, and the digest solution from the vial through the movable tube, a fluid reservoir operably coupled with the input syringe pump, a sensor configured to detect solids in the fluid withdrawn by the output syringe, and a controller configured to control the movement of the tube gripper, the input syringe pump, and the output syringe.

## Description

### BACKGROUND

The present disclosure pertains generally to autologous cell therapy. More specifically, the present disclosure relates to automated cell preparation for use in various autologous cell therapy procedures.

Autologous cell therapy (ACT) is a therapeutic intervention that uses an individual's cells, which are biopsied, cultured and expanded outside the body, the reintroduced into the donor for treatment. ACT provides for a minimization of risks associated with the use of donor tissues such as systemic immunological reactions, bio-incompatibility, and disease transmission associated with grafts or cells not cultivated from the individual. To date, ACT has been used successfully to bioengineer skin substitutes, aid wound healing, counteract chronic inflammation, treat burns and pressure ulcers, and improve postoperative healing. Further, ACT has been used to enhance the healing process of conditions such as a damaged myocardium, developing hyaline cartilage, and in the treatment of neurodegenerative diseases and other ailments that benefit from the immediate availability of a donor. The use of ACT for cosmetic enhancement or corrective surgery is also gaining recognition as a creditable form of treatment and has been shown to reduce the risk of rejection and to have longer lasting effects than conventional treatments. This form of treatment is under intense investigation with the hope that it will eventually be able to replace conventional forms of plastic surgery to improve the repair process of aging or damaged tissues.

Over the past few decades, since the bioengineering revolution, autologous cell therapy has become a rapidly evolving field. From the discovery of plasmids as vectors for bulk protein cultivation in 1973, and the production of recombinant human insulin in 1978, to the construction of the world's first artificial cornea in 1999, biosynthetic solutions to human conditions have been of great revelation and interest in the scientific industry.

One of the greatest success stories of modern medicine has been the advent of organ transplantation. As life expectancy in the developed world increases, the expiration of body tissues and organs through attrition, disease, or even trauma, is inevitable. Despite its ability to either restore a normal standard of living or extend life, organ transplantation is plagued with its fair share of problems. Almost a victim of its own success, the body's immune system can sometimes recognize the foreign entity and reject the new tissue or organ regardless of patient matching and immunosuppressive drugs. Such consequences and shortages in supply have increased interest in regenerative and autologous therapies.

Although many cells respond to signaling and stimuli in the body, they can also be cultivated outside the human body in Petri dishes and culture flasks. Tissue engineering techniques allow cell types to be grown in isolation using defined media for optimal growth. Such a capacity allows for the culture and study of cells more closely and independently of the organ from which they are a part. More impressively, this enables a small number of cells taken from an individual to be expanded outside the body and reintroduced into the donor for therapeutic intervention.

The scope for ACT is vast and has immense potential for rejuvenation. Much of this form of therapy is currently under investigation or in clinical trials. These include the treatment of limb ischemia, bone marrow mononuclear cell transplantation treatment for acute radiation victims, ischemic stroke and ischemic heart disease, cell-mediated immunotherapy after chemotherapy, autologous hematopoietic cells as targets for gene transfer to treat various blood disorders and autoimmune diseases, the use of autologous macrophages to treat acute complete spinal cord injury, cell therapy with autologous lymphocytes to treat various cancers, cell therapy for inner ear cell degeneration, and autologous serum for treatment of ocular disorders.

Some of the challenges facing any cellular transplant include, the ability of the cells to integrate and function alongside resident cells without interfering with other cells or cellular reactions. The major disadvantage of using allogeneic grafts or transplants from non-self is the possibility of systemic immunological reactions. At best, such a response could be uncomfortable and at worst life-threatening, either of which would require a lifetime of immunosuppressive drug therapy. Another concern is the risk of disease infection from animal transplants; for example, porcine endogenous retrovirus (PoERV) has caused concerns with xenotransplantation. Present in the genome of all pigs, this virus is genetically transmitted and is able to infect human cells. Concerns surround the release of the virus from the xenograft, subsequent infection of host cells, and eventual production of lymphomas.

Commercial demands for any biotechnological practice require that the process and products are cost-effective, fit for purpose, and either easily produced or accessible. Cell cultivation often employs the use of undefined components, such as animal serum, in culture media in order to accomplish desired cell growth while controlling costs. The potential risks are that animal-derived products will contaminate or alter cells, and the inability to categorically define the nature and expression of biomolecules and factors that are present. Other aspects of tissue culture being called in to question include, the use of various growth factors, extrinsic factors, such as temperature, CO2, and humidity, which may influence the metabolic activity of the cells and therefore the function, specificity, and efficacy. One significant disadvantage of ACT is that it is not immediately available for treatment in many cases. A significant lag exists between the extraction and cultivation of the cells and their use, which is dependent on the type of treatment. Ultimately, this treatment is not rapid and relies heavily upon cell growth and cultivation; any problems with this process could further delay treatment by weeks. In some cases, a temporary measure would also be required to subdue symptomatic problems and pain in the interim.

Autologous cell therapy uses a patient's cellular material to treat that patient's disease. The use of autologous cells can reduce the risks of immune rejection and disease transmission, but requires patient-unique cells to be prepared for each patient. The process includes sampling the patient's cells, and isolating, purifying, expanding, harvesting and formulating the cells. Special processes are included before expansion to genetically modify the cells. When the process is complete, the cells are returned to the patient. Currently, facilities for autologous therapies are set up to support research projects and clinical trials, and use reconfiguration of manufacturing space to produce different types of cell products, which is not a flexible arrangement in an industrial facility.

Commercial production of autologous cells is generally optimized for certain types of products. Currently, a manual biopsy digestion process includes taking the biopsy and providing it to a laboratory. In the laboratory, technicians are required to open the biopsy, manually process the biopsy, and place the biopsy in a centrifuge. Overall, what is needed is a large scale manufacturing facility that timely processes cells. However, as ACT is scaled up and automated to generalize autologous cell production, issues arise related to quality control, the uncertainties in complex propagation, time-dependence, and personalized manufacturing.

There is, therefore, a need for an automated system designed to optimize biopsy digestion to be equivalent or better than what is currently achieved using a manual process in terms of process time, media usage, and cell yield.

### SUMMARY

In accordance with some configurations, the present teachings include a system and method for extracting individual cells from a biopsy. In an aspect, the user mounts a disposable set and processes reagents into the system, adds a cap to a biopsy vial and a cell flask, and inputs the vial to the system to begin processing. The automated process includes, but is not limited to: (a) removing the biopsy transport fluid to a quality control vial, (b) washing the biopsy to remove the transport fluid and contaminants, (c) digesting the biopsy through mixing, agitation and the use of digestion solution, (d) quenching the digestion reaction with media, (e) washing the digested biopsy tissue and cells with media, and (f) transferring the digested biopsy and cells to the cell flask. In an aspect, the digestion solution is maintained at 37°C. In an aspect, the cell flask is a T25 flask. In an aspect, an external or integrated centrifugation process is used after the biopsy is washed and before the tissue is transferred to the cell flask. In an aspect, the user sterilely removes the biopsy from the system, performs centrifugation outside of the system, and then returns the biopsy to the system for continued processing.

In an aspect, an automated handling system, such as for example, a robot or robotic arm mounts the disposable set and process reagents to the system after receiving them from upstream processes on an assembly line, after digestion and quenching the automated handling system provides the biopsy vial to a centrifuge, returns the vial to the biopsy processing system when the centrifuge process is complete, and provides the flask to downstream processing equipment when the cells are harvested.

In an aspect, the system operates in an ISO class 8 space. In an aspect, capping the biopsy vial is performed in a biosafety cabinet or equivalent space. After the vial is capped, the vial forms a closed environment when adding fluid to and removing fluid from the vial. Other disposable parts include a vented cap for the cell flask and the tubing set. The input and waste vessels can be, in some configurations, commercial components. In an aspect, the system remains fully closed during operation, and allows for sterile connections to be made between the biopsy vial and the tubing set.

In an aspect, a durable pump tower controls flow through the tubing set by using dual fluidic syringes controlled by linear motion in combination with pinch valves that direct flow to the correct locations. The pump tower may include an agitation mechanism. In an aspect, the agitation mechanism is thermally controlled. In an aspect, the agitation mechanism is an orbital shaker. In an aspect, the agitation mechanism maintains the temperature of the vial while shaking during the digestion incubation step. In an aspect, the pump tower maintains the temperature of the input reagents as well as the T25 flask.

In an aspect, the system includes an interactive user interface that guides users through the cell harvesting process. The system tracks patient samples and confirms an identity of the biopsy during various stages of the cell harvesting process. In an aspect, there is a digester system for each biopsy. In an aspect, multiple digester systems are communicatively coupled, possibly through an electronic communications network. In an aspect, a database is maintained of process information for all networked digester systems.

Once the biopsy is placed into the system, the system and method automate the extraction process by, washing the biopsy, digesting the biopsy, and pumping the digested cells into a flask. The fluid path is closed so that the process can be conducted in a generalized laboratory space. In an aspect, the system includes both durable and disposable parts, for example, the tubing, solution containers, and quality control container(s) may all be disposable parts. In an aspect, the biopsy is housed in a thermally-controlled vial that has a sterile vent and integrally formed tube connection. The tube connection spans through the vial cap, having a portion, including an extension tip, within the vial and a portion outside of the vial.

The system of the present teachings includes an automated digester. In an aspect, the digester is a closed fluid transfer system that receives a biopsy, washes the biopsy tissue, digests the tissue to separate the cells by adding a digestion enzyme, and seeds the cells into a culture flask. In an aspect, digesting using the system of the present teachings is an autologous process, i.e. the system receives cells from a patient, expands and multiplies them, and returns them to the patient. In an aspect, automation employs a programmable logic controller that coordinates the motion of a plurality of syringe pumps. In an aspect, the programmable logic controller controls volumetric flows and flow rate according to sensor data. In another aspect, the programmable logic controllers control the height of the aspirate tip with respect to the bottom of a vial containing the biopsy via step-servos and according to sensor data.

The system includes a plurality of valves that control the fluid path. In an aspect, a cassette interface between the durable and disposable components is mounted into a tray that includes a face positioning tubing against which valves actuate to pinch the tubing to control fluid flow in the system. The system includes thermal controllers for the biopsy vial, the cell flask, and media bags. The programmable logic controller communicates with and controls an agitation mechanism. All these systems coordinate to move the fluid around various paths at different rates, apply wash/digest/growth fluids at different steps, and capture fluids for sampling or processing.

In an aspect, the system is controlled to execute a pre-selected recipe. The recipe can be associated with a particular biopsy, can be developed by a physician, can be developed empirically based on previous uses of the system, and/or can be developed and changed dynamically. The recipe can be encoded or associated with an RFID chip, a bar code, a QR code, for example. In an aspect, the recipe is provided by a user or another system. In an aspect, the system can generate a recipe based on the identifying information relating to the biopsy being handled, for example, if the identifying information is associated with a previously-used recipe. In an aspect, the system can begin with a basic recipe and modify the recipe as the biopsy is processed, based on sensor data.

A user interface allows entry of information associated with the biopsy. The user can be presented with the previously-used recipe, and can update any fields that might have changed from a previous use to the current use. The user can be presented with a blank form or a prompted form that can be used to provide recipe information. The user can enter recipe data into a graphic display, through a touch screen, for example. The graphic display can be a visual representation of the system, including pumps and valves, that can be manipulated through data entry into the display.

In an aspect, the system includes safety protection for the user and the patient. In an aspect, the system is closed, protecting the biopsy from contamination, as well as preventing contact with any human users of the system. The system prevents fluid contamination between patient samples. Safety protections are enabled by the biopsy vial cap and its interface to the rest of the system, which enable processing the biopsy and moving the biopsy in a closed system. When the vial is provided to the system, sensors located in the vicinity of the vial can read an identifying tag and provide that information to the controller.

Setup time, setup complexity, and cost of the system are minimized by the use of a plug-in cassette that provides the interface between the disposable components and the durable components. In an aspect, the disposable components such as tubing, syringes, the biopsy vial, the cell flask, and the waste container, are operably coupled with the durable components such as the syringe pumps, pneumatic system, and the linear actuator through the cassette. The cassette includes, for example, fluid paths, tube routing, and disposable component attachment features. In an aspect, the cassette and other components of the system are designed for manufacturing, for example, they can be injection molded, versus machined. In an aspect, the cassette is keyed to prevent improper placement in the digester system.

A system of one or more computers can be configured to perform particular operations or actions by virtue of having software, firmware, hardware, or a combination of them installed on the system that in operation, causes or cause the system to perform the actions. One or more computer programs can be configured to perform particular operations or actions by virtue of including instructions that, when executed by data processing apparatus, cause the apparatus to perform the actions. One general aspect includes a system for automated digestion of a biopsy. The system also includes a durable subsystem configured to control a flow of fluids and air to the biopsy; and a disposable subsystem configured to provide the fluids and air to the biopsy, the fluids washing and digesting the biopsy, the washing and digesting forming digested cells. Other embodiments of this aspect include corresponding computer systems, apparatus, and computer programs recorded on one or more computer storage devices, each configured to perform the actions of the methods.

Implementations may include one or more of the following features. The system as where the disposable subsystem may include: a biopsy vial holding the biopsy; a biopsy vial cap configured to accept the fluids and air through a vial tube, the vial tube configured to move within the biopsy vial; and at least one syringe pump moving the fluids to and from the biopsy vial through the vial tube, the fluids and the air moving the biopsy within the vial tube. The durable subsystem may include: at least one actuator configured to move the vial tube within the biopsy vial; an agitator configured to provide agitation to the biopsy vial, the agitation separating cells of the biopsy from each other; and a controller configured to command movement of the fluids to and from the biopsy vial. The fluids may include: digestion fluids, the digestion fluids creating digested cells from the separated cells. The fluids may include: wash fluids, the wash fluids clearing a transport fluid from the biopsy, the wash fluids preparing the biopsy for the digestion fluids. The disposable subsystem may include: a waste container configured to receive the fluids from the biopsy vial when the controller determines the fluid is to be discarded. The disposable subsystem may include: a sample container configured to receive the fluids from the biopsy vial when the controller determines the fluids are to be tested. The system as may include: at least one step-servo motor controlled by the controller, the at least one step-servo motor controlling a volume and a flow rate of the fluid. The at least one step-servo motor may include: controlling a height of the vial tube within the biopsy vial. The system as may include: at least one valve controlled by the controller, the at least one valve controlling a flow path of the fluid. The disposable subsystem may include: at least one syringe configured to accept the fluids. The disposable subsystem may include: a cell culture flask configured to receive combined digested cells after centrifugation. Implementations of the described techniques may include hardware, a method or process, or computer software on a computer-accessible medium.

One general aspect includes a method for automatically digesting cells. The method also includes receiving a biopsy including the cells and a transport fluid in a biopsy vial; attaching a cap to the biopsy vial, the cap including a vial tube, the cap forming an interior of the biopsy vial and an exterior of the biopsy vial, a position of the vial tube being adjustable, the vial tube extending from the interior to the exterior, the interior being scaled from the exterior; pumping air into the biopsy vial through the vial tube, the air moving the biopsy within the biopsy vial; pumping the transport fluid to a container; pumping wash fluid into the biopsy vial through the vial tube; agitating the biopsy vial. The method also includes pumping the wash fluid to the container; pumping a digester fluid into the biopsy vial through the vial tube, and centrifuging the digester fluid and the agitated washed cells forming digested cells. Other embodiments of this aspect include corresponding computer systems, apparatus, and computer programs recorded on one or more computer storage devices, each configured to perform the actions of the methods.

Implementations may include one or more of the following features. The method as may include: seeding a cell culture flask with the digested cells. The method as may include: incubating the seeded cells. The method as may include: providing the incubated cells to a patient. The method as may include: moving, by an actuator and grippers, the vial tube within the biopsy vial, the cap including a gripper feature, the gripper feature configured to enable the grippers to grab and release the gripper feature and move the vial tube out of and into the biopsy vial. The cap may include: vents configured to release and admit air from/to the biopsy vial. The method as the cap may include: at least one filter configured to filter the air. The cells may include: tissue from a patient. The method as may include: determining a position of the vial tube based at least on a sensor. Implementations of the described techniques may include hardware, a method or process, or computer software on a computer-accessible medium.

### BRIEF DESCRIPTION OF THE DRAWINGS

The foregoing features of the disclosure will be more readily understood by reference to the following description, taken with reference to the accompanying drawings, in which:
FIG. 1A is a schematic diagram of an exemplary configuration of the system of the present teachings;
FIG. 1B is a schematic diagram of another exemplary configuration of the system of the present teachings;
FIG. 2 is a relational diagram showing the interconnectivity of elements within the system of the present teachings;
FIG. 3 is a perspective illustration of an exemplary configuration of the system of the present teachings;
FIGS. 4A-4B are pictorial representations of a first exemplary biopsy vial cap of the system of the present teachings;
FIG. 5 is a pictorial representation of biopsies in the capped biopsy vial of the present teachings;
FIGS. 6A-6B are pictorial representations of a second exemplary biopsy vial cap of the system of the present teachings;
FIGS. 7A-7B are pictorial representations of a third exemplary biopsy vial cap of the system of the present teachings;
FIGS. 8A-8B are pictorial representations of a culture flask and a cap therefor;
FIG. 9 is a schematic block diagram of the automatic, robotically assisted, digester process of the present teachings;
FIG. 10 is an illustration of a biopsy vial handling subsystem of the present teachings;
FIGS. 11A-11B are an illustration of a fluid supply subsystem of the present teachings;
FIGS. 12A-12B are an illustration of a tubing cassette of the present teachings;
FIG. 13 is an illustration of the interior of the system of the present teachings;
FIG. 14 is a pictorial representation of exemplary component layouts of an automatic biopsy vial capping system of the present teachings;
FIG. 15 is a flowchart of the method of the present teachings for executing a process according to a recipe divided into phases;
FIG. 16 is an exemplary table of phases associated with an exemplary recipe of the present teachings; and
FIG. 17 is a schematic block diagram of an exemplary system of the present teachings.

### DETAILED DESCRIPTION

The system and method of the present teachings enables biopsy digestion in a closed system designed for achieving repeatable results, possibly as part of an assembly line. In an exemplary process, a vial containing a biopsy sample is inserted into the system, the vial is capped with a cap that includes a movable tube, and a tube gripper is engaged on the neck of the movable tube. The system moves the gripper which moves the movable tube up and down as prescribed depending upon the processing stage. In a first operational step, the system removes most of the transport fluid from the vial, leaving the biopsy tissue and a small amount of fluid. The transport fluid is removed by lowering the movable tube to the bottom of the vial and pumping air from an input syringe pump into the vial. The pumped air causes the biopsy tissue to float away from an aspiration tip at the end of the movable tube which is then be used to extract the transport fluid. An output syringe activates to extract the transport fluid from the biopsy vial. In an aspect, the extracted fluid in the output syringe is pumped to a waste container or to a sample vial. The extracted transport fluid may be deposited into a sample vial for quality control purposes. The system includes a wash cycle in which wash fluid is moved to an input syringe, the wash fluid is supplied to the biopsy vial. The biopsy vial may be agitated. In an aspect, cycles of supplying a pre-selected amount of wash fluid to the biopsy vial from the input syringe, agitating the biopsy vial, and withdrawing the wash fluid to the output syringe are repeated a pre-selected number of times. Following the wash cycles, the wash fluid is discarded and may be sent to a waste bag. A digest solution is added to the biopsy vial to break the biopsy tissue into individual cells. To assist in digesting the biopsy tissue, the digest solution may be agitated as will be described in detail below. The biopsy vial is centrifuged for a pre-selected amount of time to condense the individual cells into a cell pellet that sinks to the bottom of the vial and the digest fluid is drawn off through the movable tube. In one aspect of the system, growth solution is added to the biopsy vial containing the cell pellet. This part of the process can occur multiple times. The cell pellet and growth solution are transferred from the biopsy vial to a cell flask.

If the fluid removal process causes aspiration of the biopsy tissue into the movable tube, the system can include a recovery procedure. The recovery procedure can involve providing additional fluid to the vial from a fluid reservoir operably coupled with the input syringe. If the biopsy is aspirated along with the fluid through the biopsy vial movable tube, sensors detect the presence of solids within the fluid. The number of solids is tracked so that the system can determine if the correct number of aspirated solids is returned to the biopsy vial through the recovery procedure, and can repeat the recovery procedure if not. If the biopsy is aspirated, the system of the present teachings executes a recovery procedure. The recovery procedure is activated when sensors detect solids flowing past along with the fluids. The system of the present teachings inventories the solids that are reported and ensures, during the recovery procedure, that as many biopsies are returned to the vial as have been aspirated.

In some configurations, the system automatically transfers the biopsy vial to a centrifuge. Grabbing the vial can be enabled by, for example, robotic pick and place technology. The biopsy vial and/or cap can include a feature such as, for example, a QR code, a datamatrix code, a tier 2 bar code, and/or a 3D bar code, that can indicate to a robot the location and identity of the biopsy vial. The robot can use a vacuum, for example, to grab the vial and move it from place to place. Other possibilities include a magnetic lift or lift features built into the cap/vial.

The automated process outlined herein is enabled by at least one controller receiving data from at least one sensor. In an aspect, the controller uses information about the fluid moving from the syringe to the biopsy vial to move from one stage of the process to the next. In an aspect, this information is provided by syringe pump sensors that are used to determine the direction of fluid flow. For example, a current sensor can determine if the delivery stroke of the syringe has bottomed out, i.e. that all the fluid has been delivered from the syringe to the biopsy vial. In an aspect, a sensor is used to detect a height of the aspiration tip of the movable tube relative to the bottom of the vial. The sensor can include a laser sensor. In an aspect, an actuator provides to the controller information about whether a syringe pump is pulling fluid into the syringe from the biopsy vial or media sources, or pushing fluid out of the syringe into the biopsy vial or into waste or other output containers. In an aspect, the controller combines data from the actuator and the aspiration tip sensor to determine if an aspiration is ongoing. In an aspect, the controller can be used to count the aspirations. In an aspect, a gripper sensor is used to determine whether the grippers are open or closed. When the grippers are open, the biopsy vial can be removed, for example, to the centrifuge. When the grippers are closed, the movable tube, and thus the aspiration tip, can be displaced within the biopsy vial. In an aspect, bubble sensors are used to determine if there is air in the tubing and the amount of air contained therein. This information can be used to determine if fluid delivery has ceased.

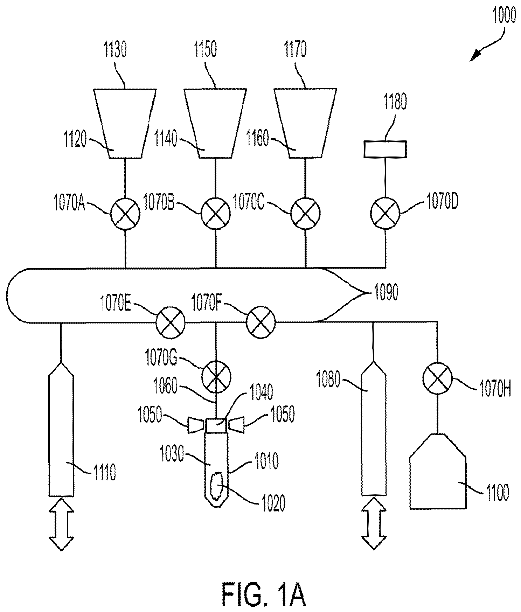

Referring now to FIG. 1A, a diagranunatic illustration of one exemplary configuration of a system 1000 in accordance with the present teachings is shown in closed form. A biopsy vial 1010 containing a biopsy tissue 1020 sample and a volume of transport fluid 1030 therein has a replacement cap 1040 affixed therero. The biopsy vial 1010 with the replacement cap 1040 is mounted to the system 1000 so that at least a portion of the replacement cap 1040 can be grabbed and operably moved as will be described below by, for example, gripper jaws 1050. A movable tube 1060 that extends through the replacement cap 1040 is fluidically connected to the system 1000 such that the biopsy vial 1010 is now connected to a closed sterile loop within the system 1000. When the processing sequence begins, valve 1070E is closed while valves 1070G and 1070F are opened. A pump 1080 is actuated to create suction in the tubing loop 1090 that operates to draw a quantity of transport fluid 1030 from the biopsy vial 1010. Preferably, substantially all of the transport fluid 1030 is withdrawn leaving only the biopsy tissue 1020 and a small amount of transport fluid 1030 in the biopsy vial 1010 for further processing by the system 1000. Valve 1070F is then closed and valve 1070H is opened and pump 1080 is actuated to eject the withdrawn transport fluid 1030 to a waste container 1100.

The system 1000, with valve 1070E closed opens valve 1070A and actuates input pump 1110 to draw a quantity of wash fluid 1120 from the wash fluid reservoir 1130. With valves 1070A and 1070F closed, and valves 1070E and 1070G open, input pump 1110 is again actuated to dispense the quantity of wash fluid 1120 into the biopsy vial 1010 to wash the biopsy tissue 1020. With valves 1070E and 1070H closed and valves 1070F and 1070G open the output pump 1080 is actuated to draw the wash fluid from the biopsy vial 1010. Valve 1070F is then closed and valve 1070H is opened so the output pump 1080 can again be actuated to dispense the withdrawn wash fluid to the waste container 1100. The washing process may be repeated for a plurality of washing cycles.

For the next step in the processing of the biopsy tissue 1020, with valve 1070B open and valves 1070E and 1070A closed, the input pump is actuated to draw a quantity of digester fluid 1140 from the digester fluid reservoir 1050. With valves 1070A, 1070B and 1070F closed, and valves 1070E and 1070G open, input pump 1110 is again actuated to dispense the quantity of digester fluid 1140 into the biopsy vial 1010 to break down the biopsy tissue 1020 into individual cells. At this point, the biopsy tissue 1020 may be agitated as will be described in more detail below to assist the digester fluid 1040 in breaking down the biopsy tissue 1020. With valves 1070E and 1070H closed and valves 1070F and 1070G open the output pump 1080 is actuated to draw the digester fluid from the biopsy vial 1010. Valve 1070F is then closed and valve 1070H is opened so the output pump 1080 can again be actuated to dispense the withdrawn digester fluid to the waste container 1100. The digesting process may be repeated for a plurality of cycles.

The biopsy vial 1010 containing the broken down biopsy tissue 1020 and some quantity of digester fluid is removed from the system 1000 and centrifuged to separate the cells of the biopsy tissue 1020 from the fluid in the biopsy vial 1010 such that the cells settle in a mass from the fluid. The centrifuged biopsy vial 1010 is then returned to the system 1000 and depending on the level of digestion achieved is advanced to the next step in the process. Should further digestion be required, the digestion steps described above may be repeated.

Once the cells of the biopsy tissue 1020 are broken down sufficiently, growth fluid is added to the biopsy vial 1010. With valve 1070C open and valves 1070E, 1070B and 1070A closed, the input pump is actuated to draw a quantity of growth fluid 1160 from the growth fluid reservoir 1170. With valves 1070A, 1070B, 1070C and 1070F closed, and valves 1070E and 1070G open, input pump 1110 is again actuated to dispense the quantity of growth fluid 1160 into the biopsy vial 1010 to aid in growing a culture of the digested cells from the biopsy tissue 1020. At this point, the biopsy tissue 1020 may be agitated as will be described in more detail below to assist in suspending the cells of the biopsy tissue 1020 in the growth fluid. The biopsy vial 1010 containing the cells of the biopsy tissue 1020 and growth fluid 1060 may again be centrifuged. Further, with valves 1070E and 1070H closed and valves 1070F and 1070G open the output pump 1080 may be actuated to draw the growth fluid from the biopsy vial 1010. Valve 1070F is then closed and valve 1070H is opened so the output pump 1080 can again be actuated to dispense the withdrawn growth fluid to the waste container 1100. The growth fluid process may be repeated for a plurality of cycles as the culture grows.

Input pump 1110 and output pump 1080 may be of any type suitable in the art. They may be cartridge pumps, vacuum pumps and/or syringe pumps. Preferably, input pump 1110 and output pump 1080 are syringe pumps that are actuated by a controller within the system 1000. Further, valves 1070A-H may be any suitable valve known in the art. Preferably, valves 1070AH are pinch valves that operate a force that compresses or pinches a closed tubing to effect closing thereof.

The tubing loop 1090 is also shown to include an air vent 1180 that is isolated by valve 1070D. At times during the process existing fluid within the tubing set 1090 may need to be purged before drawing a different type of fluid into the tubing set or dispensing a different type of fluid. In this case, valve 1070D can be opened and input pump 1110 actuated to draw in air via air vent 1080 and purge residual fluid from the tubing set 1090. The residual fluid and air can then be dispensed to the waste container 1100 as has been described.

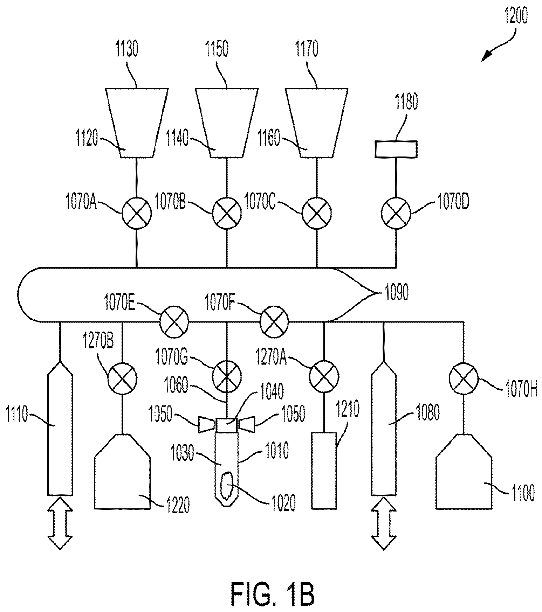

Referring now to FIG. 1B, a diagrammatic illustration of another exemplary configuration of a system 1200 in accordance with the present teachings is shown in closed form. As described above, a biopsy vial 1010 containing a biopsy tissue 1020 sample and a volume of transport fluid 1030 therein has a replacement cap 1040 affixed therero. The biopsy vial 1010 with the replacement cap 1040 is mounted to the system 1200 so that at least a portion of the replacement cap 1040 can be grabbed and operably moved as will be described below by. for example, gripper jaws 1050. A movable tube 1060 that extends through the replacement cap 1040 is fluidically connected to the system 1200 such that the biopsy vial 1010 is now connected to a closed sterile loop within the system 1200. When the processing sequence begins, valve 1070E is closed while valves 1070G and 1070F are opened. A pump 1080 is actuated to create suction in the tubing loop 1090 that operates to draw a quantity of transport fluid 1030 from the biopsy vial 1010. Preferably, substantially all of the transport fluid 1030 is withdrawn leaving only the biopsy tissue 1020 and a small amount of transport fluid 1030 in the biopsy vial 1010 for further processing by the system 1000. Valve 1070F is then closed and valve 1270A is opened and pump 1080 is actuated to eject the withdrawn transport fluid 1030 to a quality control container 1210. In this manner the withdrawn transport fluid can be saved for further quality control testing and/or to determine if the original biopsy vial 1010 was contaminated prior to processing by the system 1200.

The system 1200, proceeds with the wash and digestion steps as described above. At the end of the process of adding growth fluid 1160, the cells from the digested biopsy sample 1020, suspended in growth fluid 1160 are transferred to a cell growth flask 1220. With valve 1070G and 1070E open and the remaining valves closed, the input pump is actuated to draw a quantity of growth fluid 1160 with the cells from the digested biopsy sample 1020 suspended therein from the biopsy vial 1010. With valve 1270B open and the remaining valves closed, input pump 1110 is again actuated to dispense the quantity of growth fluid 1160 with the cells from the digested biopsy sample 1020 suspended therein into the cell growth flask 1220 where the culture is left to grow. At this point the culture is monitored and additional growth fluid 1160 may be added to the cell growth flask 1220 as needed.

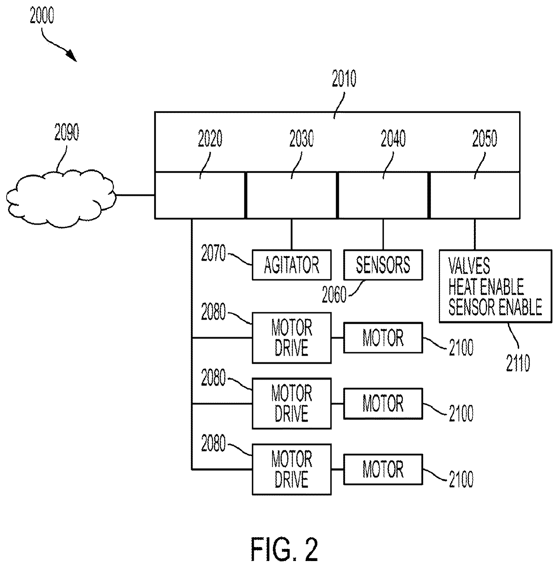

Referring now to FIG. 2, the system of the present disclosure includes a control module 2000 which is illustrated in a schematic block diagram. In an aspect, the process is instructed, carried out, controlled, and/or monitored by a Programmable Logic Controller (PLC) 2010. The PLC 2010 includes a processor that interfaces with a dual channel Ethernet module 2020, at least one serial I/O bus 2030, at least one analog I/O module 2040, and at least one digital I/O module 2050. The at least one analog I/O module 2040, and at least one digital I/O module 2050 receive data from sensors 2060, such as for example, bubble sensors, lasers, and temperature sensors and transfers the received data to the PLC 2010 for further use and processing. At least one agitator 2070 which communicates with and is controlled by PLC 2010 through serial I/O bus 2030. In an aspect, the main form of communication between PLC 2010 and at least one motor drive(s) 2080 is EthernetlP through Ethernet module 2020. In an aspect, PLC 2010 also communicates with the internet/cloud 2090 through a second, isolated Ethernet channel in Ethernet module 2020, thus insulating the machine components from direct contact with the internet 2090. PLC 2010 interfaces with motor drive(s) 2080, and sends commands to motor drive(s) 2080 which in turn command motors 2100. Other operable devices 2110 such as valves, heaters, operable sensors, etc interface with the PLC 2010 via the at least one digital I/O module 2050. Other forms of connectivity and connectivity maps are contemplated by the present teachings. The various I/O modules 2020, 2030, 2040 and 2050 are connected to PLC 2010 enabling communications among the various components of the system through a backplane. In an aspect, the machine components are hardwired into the system. In an aspect, PLC 2010 may communicate with all components of the system through a communications protocol over various forms of communication links including, but not limited to, wide area network, local area network, wifi, and BLUETOOTH^{®} communications means.

Continuing to refer to FIG. 2, in an aspect, the logic executed by PLC 2010 is arranged into tasks, each having a scan time. In an aspect, safety and motion control tasks have a relatively fast scan time, for example, 10 ms. Sensors, in general, have a relatively moderate scan time, for example, 250 ms, and system level logic can operate with a relatively slow scan time, for example, 500 ms. In accordance with ISA-88 Batch Control, to carry out procedures like adding fluid or heating, the system includes various control modules, equipment modules, and phases. Control modules manage the actions of, for example, motors, agitators, valves, bubble sensors, aspirate sensor, and heaters. Motor control modules are responsible for enabling/disabling motor drive fault handling logic, homing, and moving. The main sequence in an exemplary control module reacts to a fluid/air move request, determines a "dose" for either fluid or air movement, and manages the move. In an aspect, fluid/air movement is discontinued when the desired volume is reached, the maximum move allowance is met, when an error occurs, or when a synchronized fluid movement is completed. In an aspect, the agitator control module polls the agitator device with a set of queries and sends command requests. In an aspect, the valve control modules monitor the feedback of each of the valve(s) (if available) and report an error if a valve does not perform its commanded action in a given time. In an aspect, the bubble sensor control modules manage multiple bubble sensors that enable dose totalizing. In an aspect, the temperature control modules manage multiple thermal regulators that provide temperature adjustment to the media and cells located throughout the system. In an aspect, analog input and feedback control, for example, from a proportional-integral-derivative (PID) controller, are input to the temperature control modules. Other control modules and distribution of tasks are contemplated by the present teachings. The system of the present teaching is not limited to the exemplary configuration described herein. Equipment modules sequence the various actions of the control modules to perform various tasks.

Continuing to refer to FIG. 2, PLC 2010 execution is organized into phases according to, for example, ISA-88 Batch Control. Phases are the lowest level of procedural element in the procedural control model. Procedural control refers to control that directs equipment-oriented actions to take place in an ordered sequence in order to carry out process-oriented tasks. The procedural states for a given phase can include aborting, holding, resetting, restarting, running and stopping. phases sequence the equipment modules to achieve a desired function. For example, a desired function of transferring fluid includes the steps of configuring the valves using an equipment module associated with a manifold, configuring the biopsy vial using an equipment module associated with the biopsy vial, and modifying the biopsy vial movable tube position using an aspirate equipment module. In an aspect, the sequencing of phases is controlled by a recipe.

The recipe can be preset, dynamically varying based on, for example, sensor data, and/or set by a user. In an aspect, the recipe can be modified by a commercial recipe editor. In an aspect, parameters with settings that can be varied are associated with pumps, the biopsy vial movable tube, the manifold, the agitator, the agitator heater, phases, pump transfers, motor initialization, the media heaters. the cell culture flask heater, equipment phase failures, and alarms, among other settings. For example, the biopsy vial can include an identification means that is manually or automatically accessed when, for example, the vial is installed, either manually or automatcally (e.g. via robot) into the system. The identification can be used, among other things, to access a preset recipe. The idenfication can include, but is not limited to including, a QR code, a bar code, a 3D bar code, or another other form of unique identification. In addition to recording the identification of the biopsy vial, in certain phases, the system can verify the identify of the biopsy vial and raise an alert if the biopsy vial does not contain an identification known to the system. In an aspect, the identification can be manually accessed, for example, by a handheld barcode scanner, connected by wireless USB to a user interface device. In an aspect, the system includes a scanner that is automatically activated when the biopsy vial is mounted into the system.

Continuing to refer to FIG. 2, PLC logic (a) provides component identification and network addresses for various components of the system, including users, groups, and permissions; (b) provides coordination of software phases on the PLC and the phase inputs and outputs that can then be used to sequence phases, allowing users to create custom recipes without alterations to the software on the PLC; (c) provides a method to insert manual operations into the sequence, for example, loading or unloading a cassette, that captures the actions and signature of the user; (d) provides logging; (e) provides supporting databases such as, but not limited to, historian, recipes, alarms, and events; and (f) provides a human-machine interface (HMI) across a distributed network to interface with the components of the system and possibly with other systems.

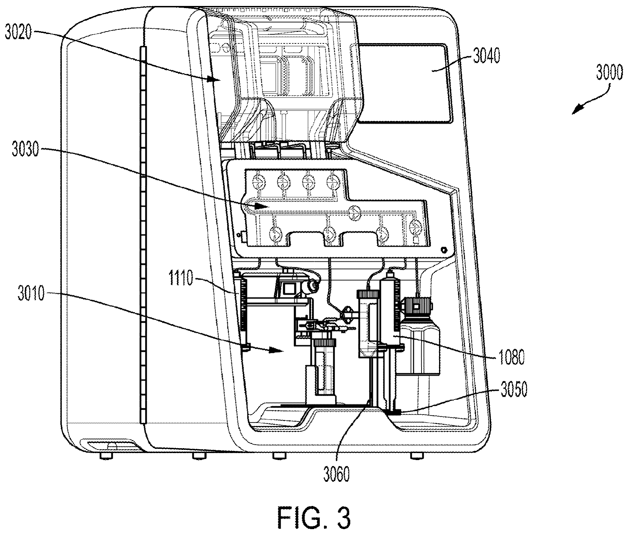

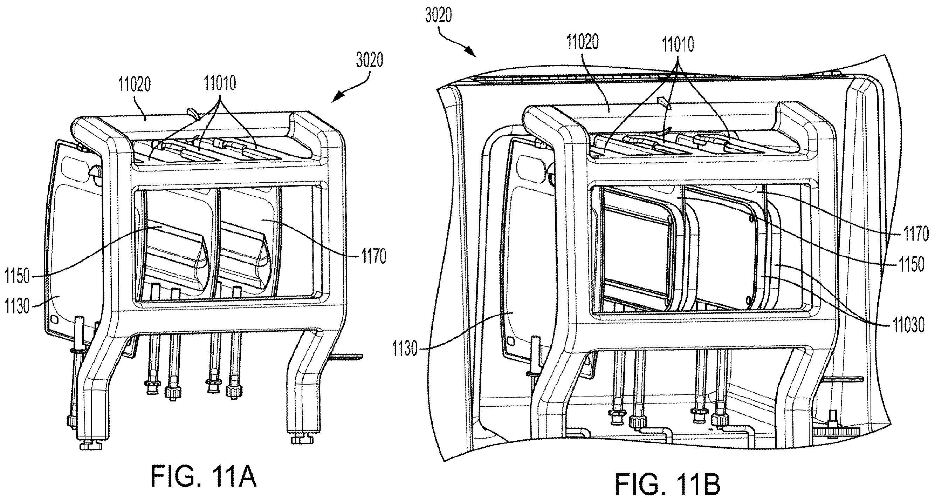

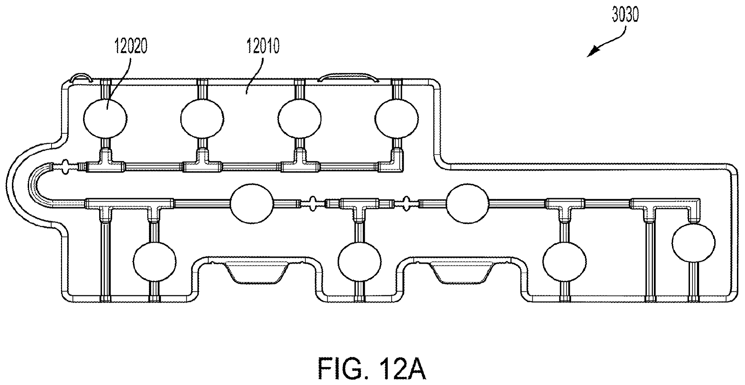

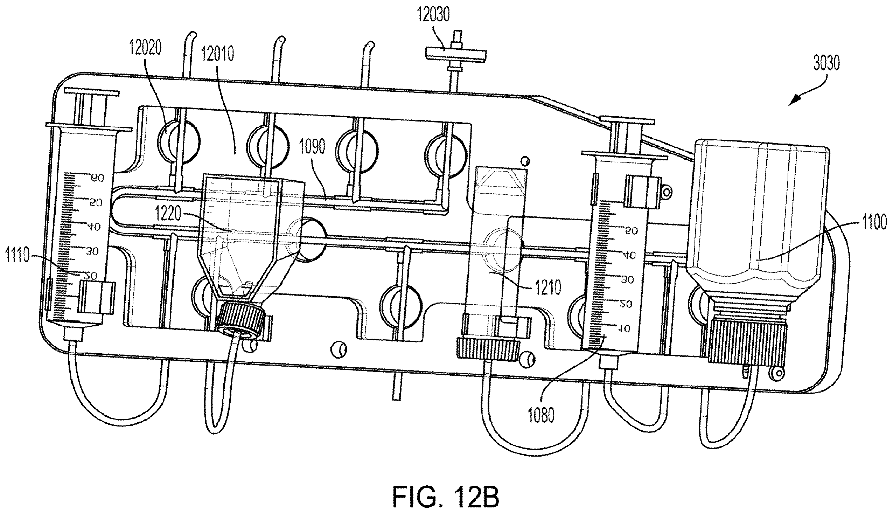

Turning now to FIG. 3 and exemplary embodiment of the system 3000 is shown. The system 3000 includes a biopsy vial subsystem 3010 (further illustrated in detail at FIG, 10), a fluid supply subsystem 3020 (further illustrated in detail at FIG. 11), and a tubing cassette 3030 (further illustrated in detail at FIG. 12) containing the tubing 1090 extending between the fluid supply subsystem 3020 and the biopsy vial subsystem 3010. A user interface 3040 is provided for interfacing with the system 3000 for adjusting operating parameters, controlling the process and providing reporting on the process. The user interface may be a touch screen interface, manual controls or combinations thereof. The user interface 3040 includes user communications facility. Options for user communications facility include, for example, but not limited to, a touchscreen for user control, a dashboard camera for recording actions with respect to the system and automatically transmitting them to a monitoring facility, and audio/video conversational interface.

Brackets 3050 that interface with the linear actuator 3060 and displace the plungers of the syringe pumps 1080 and 1110 are engaged with the syringe plungers. In an aspect, home position for the plungers is when they are in topmost position. The other extreme plunger position is fully bottomed out in the syringe, the position in which the plunger arrives in the cassette. The system controller uses step-servo motor encoder data to control the syringe plunger position. The volume of fluid pumped into/out of the syringe is calculated based on the steps the motor takes and the diameter of the syringe. Such a process is used to control fluid delivery rate. In an aspect, the transport fluid in which the biopsy arrives is pumped, after the disposable components are in place, into the sample vial to be evaluated for contamination. Spent fluids, for example the transport fluid after evaluation, are pumped into a waste container. In an aspect, the waste container includes a barbed waste container cap. To determine when the syringe is homed, that is, when the bracket is pushed to its height extreme, the current in the actuator is read. In an aspect, when the current takes on a pre-selected value, for example, 10 amps, the syringe is fully homed. In an aspect, other means can be used to determine the syringe home position, for example, a Hall sensor or other kind of sensor could be used to examine the syringe's physical location. In an aspect, a bubble sensor is used to establish exactly how much fluid is moving through the fluid path. Syringes facing upwards displace air first which can be sensed and used for fluid volume control. In an aspect, the system includes a venting sequence to purge the tubing of air.

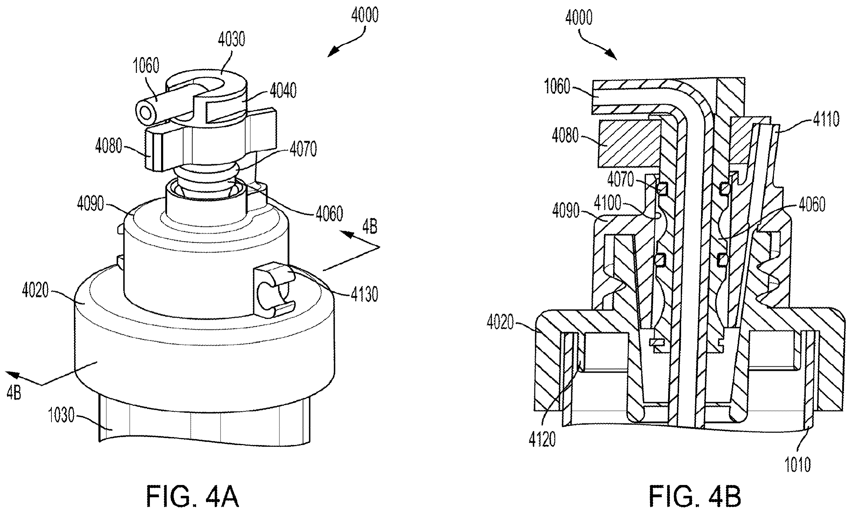

Referring now to Figs. 4A and 4B, a first configuration of the biopsy vial cap 4000 of the present teachings is shown and illustrated. The cap 4000 is used to replace a conventional cap found on a conventional externally-threaded biopsy vial 1010 in order to provide aspiration capabilities according to the present teachings. One configuration of the cap 4000 of the present teachings includes base cap 4020, top cap 4030 configured with gripper indentation 4040 to accommodate a gripper (shown elsewhere) that will enable the movable tube 1060 to move in a linear fashion in an up and down direction within biopsy vial 1010. The cap 4000 further may include an inner bellows 4060 configured to position o-rings 4070, spacer 4080, and threaded upper cap 4090. Spacer 4080 provides a limit stop to set the furthest extent to which the movable tube 1060 can be extended into the biopsy vial 1010. O-rings 4070 create a seal against a passageway 4100 in the upper cap 4090 and create a closed system, i.e. o-rings 4070 enable a sterile boundary between the environment and the interior of the biopsy vial 1010. Movable tube 1060 is moved up and down by gripper arms 1050 that are components of the system of the present teachings described herein. In an aspect, the movable tube 1060 is press fit into the inner portion of bellows 4060. In an aspect, movable tube 1060 is a bent tube that travels up and down into and out of biopsy vial 1010 through bellows 4060 that maintains a closed barrier. In an aspect, port 4110 allows air to be vented into and out of biopsy vial 1010 and may include a vent filter (not shown) that filters incoming and outgoing air and maintains a sterile boundary between the environment and the interior of biopsy vial 1010. Plug seal 4120 provides an environmental barrier for base cap 4020. Plug seal 4120 extends from the inner surface of base cap 4020 to the upper surface of biopsy vial 1010 and assists in sealing with the top edge thereof. In an aspect, tube management feature 4130 is provided to accommodate tubing required for the centrifuge process. Tube management feature 4130 can be operably coupled with, for example, but not limited to, threaded upper cap 4090, or any other place that is convenient to place tubing to enable the operation of the system of the present teachings.

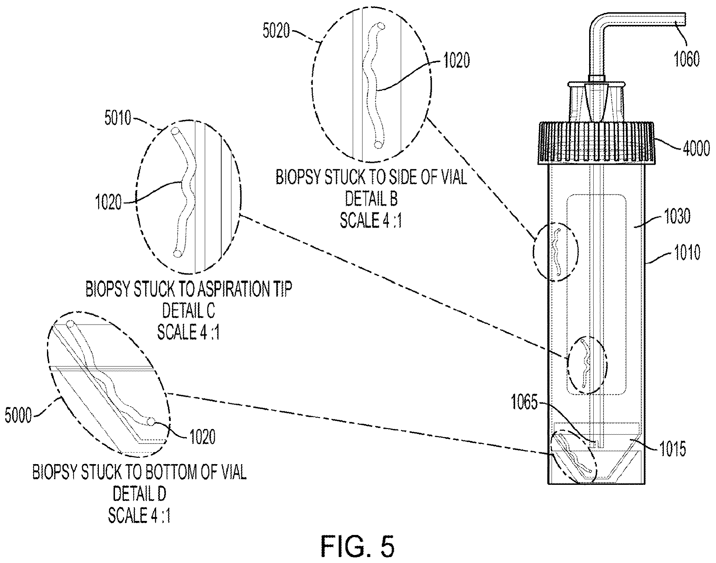

Referring to Figs. 4A and 4B in combination with Fig. 5, biopsy vial 1010 initially contains transport fluid 1030 and one or more biopsy tissue samples 1020 floating within the transport fluid at various locations in biopsy vial 1010 are shown. Biopsy vial 1010 can arrive with biopsy tissue samples 1020 located anywhere in the vial such as, for example location 5000 near floor 1015 of vial 1010, location 5010 adhering to the movable tube 1060, and/or location 5020 adhering to the side of vial 10107. In the system of the present teachings, air is introduced to vial 1010 through the movable tube 1060 to move any biopsy tissue samples 1020 away from bottom tip 1065 of the movable tube 1060. Fluid can then be introduced or removed through the movable tube 1060 without injuring biopsy tissue samples 1020 because tissue samples 1020 will have been moved away from bottom tip 1056 by the previously-introduced air.

The vial cap 4000 of the present teachings is initially installed as a replacement cap upon biopsy vial 1030 with an aspiration tip at the distal end of the movable tube 1060 located in a relatively high position with respect to the base of biopsy vial 1010 in order to add air or add/remove fluid without affecting the biopsy(ies). To extract fluid from vial 1010, air is used to move the biopsy tissue sample away from the lower end 1065 of the movable tube 1060 near the bottom of vial 1010, of movable tube 1060 in order to protect the biopsy from injury. A linear actuator is used to move movable tube 1060. After the biopsy is nudged away from the base of movable tube 1060, movable tube 1060 is moved towards the bottom of biopsy vial 1010, and slightly away from the bottom 1015 of biopsy vial 1010. Transport fluid 1030 is extracted from biopsy vial 1010 and pumped, for example, to a quality control vial or a syringe or a waste container. Wash solution is admitted to biopsy vial 1010 through movable tube 1060 to wash the biopsy, then aspirated out from biopsy vial 1010. This process can be completed in multiple iterations, each time the wash fluid is pumped to waste, for example. In an aspect, a sensor monitoring flow through the movable tube 1060 signals if the biopsy has been aspirated, triggering a sequence to return the biopsy to biopsy vial 1010.

Continuing to refer to Figs. 4A and 4B, in an aspect, digesting of the biopsy includes breaking down the protein scaffolding so the individual cells can float and separate from one another. A digesting enzyme can be used for this purpose. After the biopsy is washed, the digestive enzyme is moved from a source bag to a pump, preferably a syringe pump, that then transfers the digestive enzyme into biopsy vial 1010 through movable tube 1060. When the biopsy has reached a desired state, gripper arms are released from movable tube 1060, which releases biopsy vial 1010 from movement constraints. In the unconstrained state, biopsy vial 1010 can be agitated for a pre-selected amount of time to separate the cells from each other. Agitation of the biopsy sample in the digestive enzyme may also be assisted by agitating the movable tube 1060 in an up and down fashion. Further, in an aspect, agitation may be accomplished by drawing digestive enzymes contain partially broken down biopsy tissue up into and ejecting them back out of the movable tube 1060. As stated above, a sensor monitoring flow through the movable tube 1060 signals if the biopsy has been aspirated, triggering a sequence to return the biopsy to biopsy vial 1010.

At a pre-selected point in the separation process, it is determined that the cells are sufficiently separated. To avoid damage to the cells, agitation of the cells is ceased and growth media is moved into a syringe and pumped into biopsy vial 1010 through movable tube 1060 to mix with the digestive enzyme. One or more centrifuge cycles can take place at this time. In an aspect, an operator manually moves biopsy vial 1010 to and from the centrifuge. In an aspect, a robot controlled by a controller or, for example, an assembly line controller, issues commands to move biopsy vial 1010 to and from the centrifuge. In an aspect, multiple digester systems surround the centrifuge, enabling the robot to pick biopsy vial(s) 1010 from each of the systems and place them into the centrifuge. In an aspect, the centrifuge is integral to the biopsy system of the present teachings, enabling the biopsy to be moved from the biopsy vial to the centrifuge and back through fluid transfer, as described herein. Following the centrifuge cycle(s), fluid is removed from biopsy vial 1010, growth media are added, and the cells and growth media are mixed and moved to a cell flask.

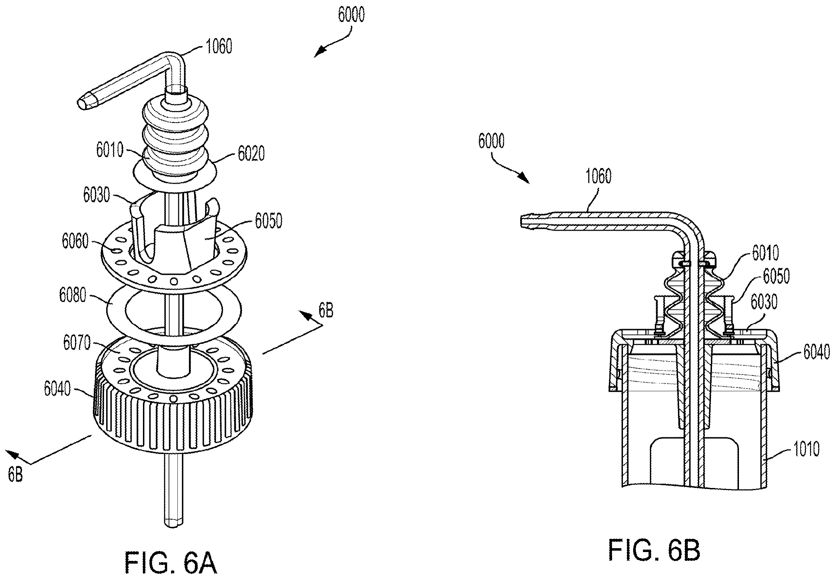

Referring now to Figs. 6A-6B, shown is a second configuration of biopsy vial cap 6000 of the present teachings. In FIG. 6A, cap 6000 replaces a conventional cap found on a conventional externally-threaded biopsy vial. In an aspect, cap 6000 utilizes bellows 6010 to assist in activating the system and sealing biopsy vial 1010 from the environment. Bellows 6010 can include any number of folds. here shown having three folds and bellows base 6020, which is larger than the folds and seals the contents of biopsy vial 1010 to/from the environment. In an aspect, cap 6000 includes base cap 6030, top cap 6040 configured with mating part 6050 that accommodates a gripping mechanism as described herein. In an aspect, both base cap 6030 and top cap 6040 include openings 6060 and 6070 which cooperatively provide venting for biopsy vial 1010. In an aspect, openings 6060 and 6070 be positioned to receive and retain a filter 6080 that filters the air that enters and leaves biopsy vial 1010, and maintains a sterile boundary between the environment and the interior of biopsy vial 1010. In an aspect, openings 6060 and 6070 are shaped circularly and/or ovular, but can take any shape, and align with each other across filter 6080. Movable tube 1060 is moved up and down by gripper arms (shown elsewhere) that are components of the system of the present teachings described herein. In an aspect, bellows 6010 may include a latching mechanism to retain movable tube 1060 at a pre-selected height during various parts of the process in which it is desired so that the position of movable tube 1060 remains substantially fixed. In an aspect, movable tube 1060 is press fit into the inner portion of the top of bellows 6010. In an aspect, movable tube 1060 is a bent tube that travels up and down into and out of biopsy vial 1010 through bellows 6010 while maintaining a closed barrier between the contents of the biopsy vial and the environment.

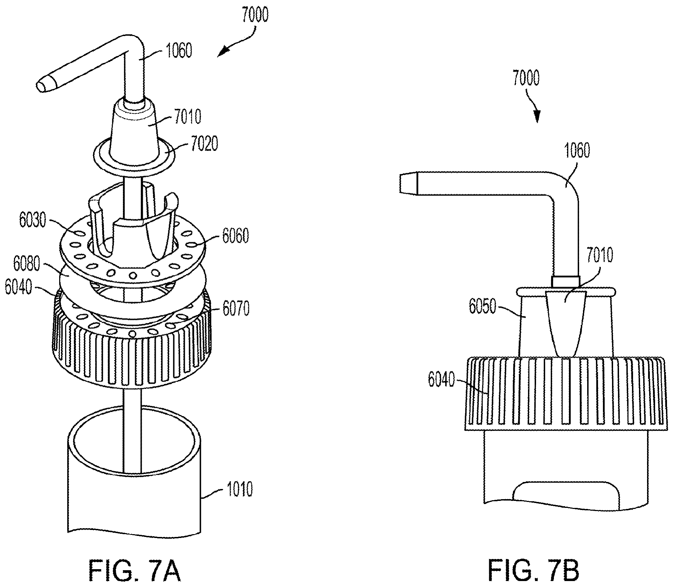

Referring now to Figs. 7A and 7B, another configuration of the cap of the present teachings, cap 7000, is shown. In this configuration, the bellows of the previous embodiments is replaced by interface component 7010 that seals the space between base cap 6040, top cap 6030, on the lower edge 7020 of interface component 7010, while providing a friction fit on tube 1060 at the upper edge of interface component 7010. Vents 6060 and 6070 and filter 6080 ensure that the system is not over-pressured. In an aspect, the filter pore size of around .22µ prevents contaminants from entering vial 1010, while allowing air to escape. In an aspect, filter 6080 is located between the weld between base cap 6040 and top cap 6030, forming a hermetic seal.

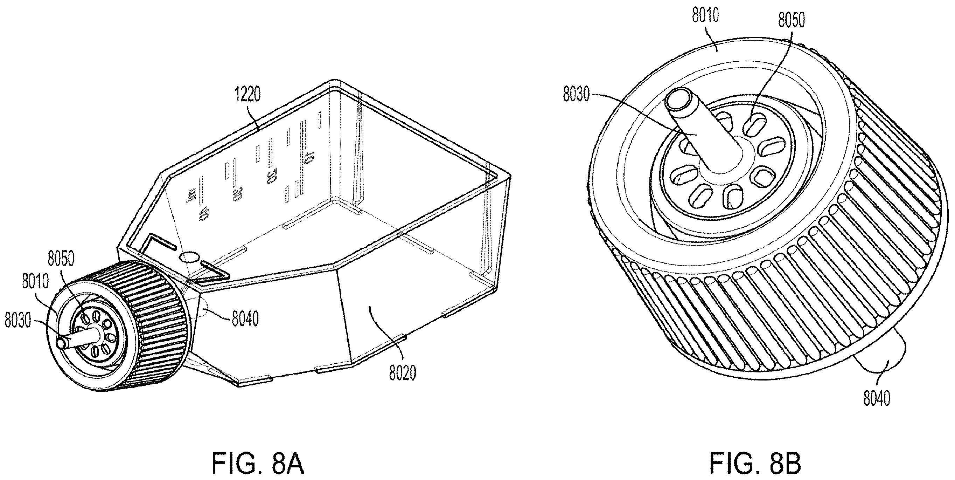

Referring now to Figs. 8A and 8B, shown is cell culture flask 1220. Cell culture flask 1220 can be any container that can operably couple with cell culture cap 8010 at the neck of cell culture flask 1220. The purpose of cap 8010 is to direct fluid and cells onto the bed 8020 of flask 1220. In an aspect, cap 8010 includes inlet 8030 and port 8040 that directs fluid to the bed 8020 of flask 1220 where a coating of protein helps cells to bond and promotes their growth. Other orientations of cap 8010, and thus port 8040, are contemplated by the present teachings. For example, port 8040 could be oriented to discharge cells onto one of the other sides of flask 1220, or to the floor of flask 1220, all of which could be considered the bed of flask 1220. Cap 8010 includes venting mechanism 8050 to enable air to enter and escape from flask 1220. In an aspect, a filter as described in the above figures with respect to the biopsy vial cap, can be included in cap 8010 as described herein.

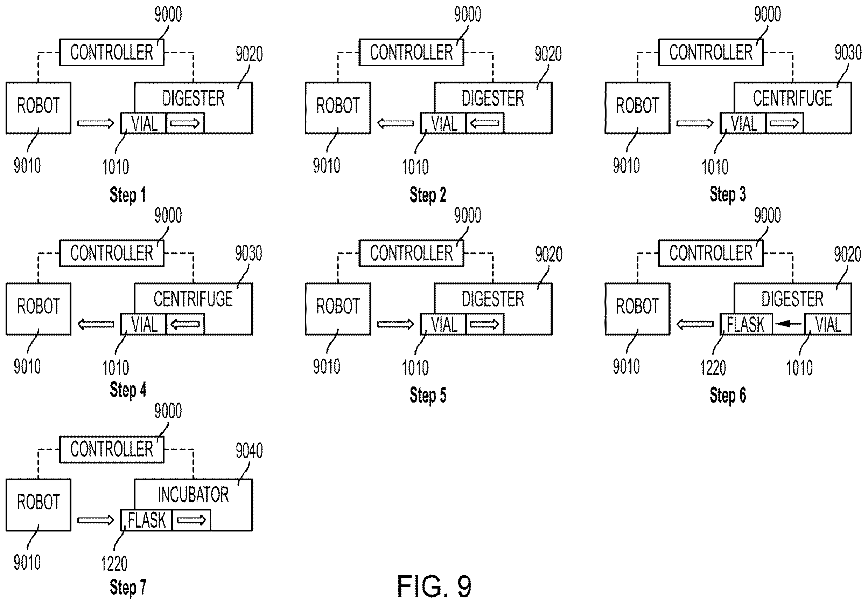

Referring now to FIG. 9, an exemplary assembly line process implementing the present teachings includes seven steps, but could have more or fewer steps as described herein. The steps are under the control of controller 9000 which is operably coupled with robot 9010, digester 9020, centrifuge 9030 and incubator 9040. In an aspect, a single robot carries out moving vial 1010 and cell culture flask 1220 from one device (digester, centrifuge, and incubator) to another, and is therefore located centrally to the devices. In an aspect, each of the devices is located along an assembly line, and multiple robots carry out vial 1010 and cell culture flask 1220 movement. In step (1), robot 9010 accesses vial 9050 and moves vial 1010 into digester 9020, which has been pre-loaded with disposable components, using an easy-mount mechanism such as a cassette. When the cassette is used, the disposable components and cassette can arrive as a package and are together aligned with and connected to the durable components of the system of the present teachings. When the cassette is not used, each component is tubed to the durable components. In either case, disposable components such as syringes, cell culture flask 1220, a sample container, fluid source containers, and a waste container are fluidically coupled with tubing. The fluid in the system is driven by valves that permit or inhibit flow through the tubing, based on commands from controller 9000. In an aspect, the centrifuge and the incubator can be fluidically coupled with the digester, obviating the need for robot 9000 to move vial 1010 from one device to another. In such a system, robot 9010 can move vial 1010 into digester 9020, and move culture contained in the flask 1220 from incubator 9040 onto another station along an assembly line.

Continuing to refer to FIG. 9, in step (2), when controller 9000 determines that the contents of vial 1010 have been prepared for centrifugation, robot 9010 retrieves vial 1010 from digester 9020, and in step (3) supplies vial 1010 to centrifuge 9030. To prepare the contents for centrifugation, digester 9020 moves the biopsy tissue away from a movable tube, the movable tube being admitted to vial 1010 by the vial cap of the present teachings, by supplying air through the movable tube into vial 1010. The air moves the biopsy tissue away from the movable tube so that fluid can enter vial 1010 without injuring the biopsy tissue. Fluid is admitted that can wash and nourish the biopsy, and the biopsy can be agitated to separate the cells from each other and from the surfaces of vial 1010. After the pre-selected preparation process completes, in step (3), vial 1010 is placed, by robot 9010, into centrifuge 9030. When the centrifuge process is complete, step (4) in the process is initiated by controller 965. In step (4), robot 9010 moves vial 1010 from centrifuge 9030. At this step, the biopsy should be a blob of cells floating in vial 1010. In step (5), robot 9010 moves vial 1010 back to digester 9020 where vial 1010 is fluidically coupled with, among other components, cell culture flask 1220. Digester 9020 executes commands from controller 9000 to remove the blob of cells from vial 1010 through the movable tube. In step (6), controller 9000 commands digester 9020 to move the cells from vial 1010 to cell culture flask 1220. The removed cells flow through tubes that operably couple vial 1010 and culture flask 1220. In step (6), controller 9000 commands robot 9010 to move cell culture flask 1220 from digester 9020 when the process of moving the cells from vial 1010 to cell culture flask 1220 is complete. In step (7), controller 9000 commands robot 9010 to move culture cell flask 1220 to incubator 9040.

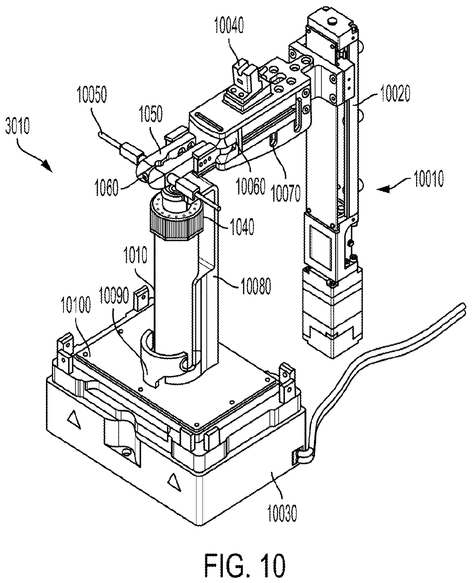

Referring now to FIG. 10, shown is the biopsy vial subsystem 3010. The biopsy vial subsystem 3010 is configured to enable safe and repeatable separation of tissue from the fluid surrounding it. The biopsy vial subsystem includes biopsy vial 1010, biopsy vial cap 1040, biopsy vial movable tube 1060, a gripper subsystem 10010, actuator 10020, sensors, and agitator 10030. Biopsy vial cap 1040 is designed to receive air that enables the biopsy to float away from the aspiration tip at the interior end of the movable tube 1060, and enables the aspiration tip to add and extract fluid from the biopsy vial 1010. Bubble sensor 10040 provides data that, at least in part, controls dose totalizing by determining if there is any fluid left in the tubing after valves have set the fluid path and fluid has been pumped along the path. The gripper subsystem 10010 includes tube gripper jaws 1050, movable tube displacement sensors 10050, gripper mechanism 10060, and gripper bracket/tube routing channel 10070. Gripper bracket/tube routing channel 10070 is a structure that holds the electronic coupling mechanism between actuator 10020 and gripper mechanism 10060. Gripper mechanism 10060 translates actuator 10020 force into movement of gripper jaws 1050. Movable tube sensors 10050 report the position of the movable tube 1060 to a controller (not shown) which provides commands to actuator 10020 based at least on a desired timing of events with respect to the biopsy. Biopsy vial 1010 is mounted in holders 10080/10090 upon platform 10100. Coupled with platform 10100 is agitator 10030. Holders 10080/10090 are positioned around biopsy vial 1010 in such a way that movement of biopsy vial 1010 is permitted. Agitator 10030, which can include an orbital shaker, provides that movement, and the movement enables the biopsy within biopsy vial 1010 to be separated into cells. In an aspect, thermal control of the contents of biopsy vial 1010 is at least in part accomplished by a thermal plate associated with agitator 10030 that adjusts the temperature of thermal conducting holder 10080 and the contents of biopsy vial 1010 through conduction.

Gripper jaws 1050 surround and grab, under pneumatic control, biopsy vial movable tube 1060. The sensors 10050 are preferably laser sensors in the exemplary configuration that includes a sensor head and two spot thru-beams monitoring the position of biopsy vial movable tube 1060. In an aspect, the laser sensor detects the presence of solids among the liquid passing through biopsy vial movable tube 1060. In an aspect, the solid is the biopsy itself. When a solid is detected, a counter is triggered. Further, the counter is incremented, for example, when a solid is detected leaving the biopsy vial as fluid is extracted from the biopsy vial, and decremented when a solid is detected returning to the biopsy vial as fluids are supplied to the biopsy vial. When the fluid exchange process is complete between the biopsy vial and other fluid containers (vials, syringes, etc.) in the system, the counter should be zero. If the counter is non-zero, a recovery procedure is activated. The recovery procedure includes pushing all the fluid from the fluid container to which fluid had been transferred into the biopsy vial and recheck the counter. If the counter is non-zero, air is pushed into the biopsy vial. If the counter is still non-zero, the recovery process is restarted. If the recovery process executes unsuccessfully for a pre-selected number of iterations, an alert is raised. Other types of sensors are contemplated by the present teachings. Sensors 10050, for example, determine when gripper jaws 1050 are in position to close on movable tube 1060. Sensors 10050 can include non-contact proximity sensors such as, but not limited to, photoelectric sensors such as, for example, thru-beam sensors, such as laser sensors. Actuator 10020 moves gripper jaws 1050 in closed position to grip movable tube 1060 and move it into and out of biopsy vial 1010. Grippers 1050 move up and down in slit along actuator 10020.

Referring now to FIGS. 11A and 11B, the fluid supply subsystem 3020 is shown. Fluid is supplied to the syringe pumps of the system for various reasons. In an aspect, fluid bags 1130/1150/1170 can be filled with different types of fluid that can be individually supplied as necessary. In an aspect, the different types of fluids can be mixed, for example during a wash cycle, when such a mixture is called for. Each of fluid bags 1130/1150/1170 is mounted upon fluid bag mounts 11010, which can be constructed in the same or different geometries to accommodate their positions in fluid bag housing 11020. Thermal control of the fluid can be accomplished by, for example, thermal control elements 11030 mounted within fluid bag housing 11020. In an aspect, thermal control elements 11030 may be mounted beside each fluid bag 1130/1150/1170. Fluids are maintained at a desired temperature within the fluid supply subsystem 3020 which is a thermally-controlled enclosure. Fluid is delivered to the tubing system from the fluid bags 1130/1150/1170. The temperature set point of the thermally-controlled enclosure can be adjusted to the desired temperature by a controller (not shown) that receives current fluid temperature data from, for example, but not limited to, in-path, in-bag, and/or remote sensors. The in-path sensors can include non-contact sensors mounted on or near the tubing connecting fluid bags 1130/1150/1170 with cassette 3030 and/or any other tubing along the line between cassette 3030 and the fluid's final destination.

The fluid supply subsystem 1030 is preferably configured as a cartridge for easy installation and removal to/from the system. Tubing at the base of the fluid bags 1130/1150/1170 preferably include releasable connectors to allow interfacing with the tubing set as will be described below.

Referring now to FIGS. 12A and 12B, a tubing cassette 3030 is shown illustrating valve layouts and vessels of an exemplary fluid delivery configuration of the present teachings. The exemplary configuration is a closed automated fluid delivery system utilizing syringe pumps and pinch valves. Various fluids such as wash, growth media, and digest media are stored in media receptacles. A base cassette 12010 is provided to receive and support the fluid supply subsystem and has openings 12020 there through across which the closed loop tubing 1090 extends such that the tubing 1090 is positioned over and accessible to the pinch valves to act thereon. It is of note that the tubing cassette 3030 is shown in a preloaded position before installation onto the system such that the various components are installed in a stowed position retained against the base cassette 12010. Cassette 12010, a disposable component of the system, is secured to and removable from a cassette ledge in the system.

FIG 12B illustrates the disposable components installed and arriving on the tubing cassette 3030, and in their operational locations. Syringe pumps 1080 and 1110, QC/sample container 1210, cell culture flask 1220, and waste container 1100 are fluidically coupled by cassette 3030 in a tray assembly, as described herein. Fewer or more fluid containers can be included in the system. In an aspect, a biopsy vial 1010 is operably coupled with a closed tubing set 1090 and means to deliver fluid through a cassette. Included are a flask 1220 to which processed cells are delivered and a biopsy vial 1010 containing the tissue sample and transport fluid. When syringe pumps 1080 and 1110 are purged of air, the air exits the system through an air filter 12030 to prevent environmental contamination. Various fluids as discussed in detail above are pumped from the fluid supply subsystem 3020 into biopsy vial 1010 via syringe pump 1110. Air exiting the system is cleaned of contaminants, maintaining environmental isolation from harmful substances. Air entering the system is cleaned of contaminants as well. In an aspect, during certain processes, the system tubing is purged of residual fluid. This is done by opening a vent, drawing in air, pushing air through the tubing, and clearing the tubing. The drawn in air passes through filter 12030 having a pore size selected to provide relatively clean air to the system. Cells are moved from biopsy vial 1010 into culture cells flask 1220 (following centrifugation). Spent media is pumped into waste receptacle 1100 and/or QC vial 1210.

Alternative configurations of the system of the present teachings are contemplated that simply provide different arrangements of the same components and using the same operational principal. For example, relatively lateral placement of the components enables a relatively small footprint of the system of the present teachings. An exception to the lateral placement is that the waste receptacle may be positioned above the QC vial and the processed cells flask. In other configurations, some of the components are situated to take advantage of gravity to help to move the fluid from place to place. Specifically, media bags may be situated above syringe pumps and biopsy vial. In other configurations processed cell flask, QC vial, and waste receptacle may be positioned relatively laterally with respect to each other and above biopsy vial.

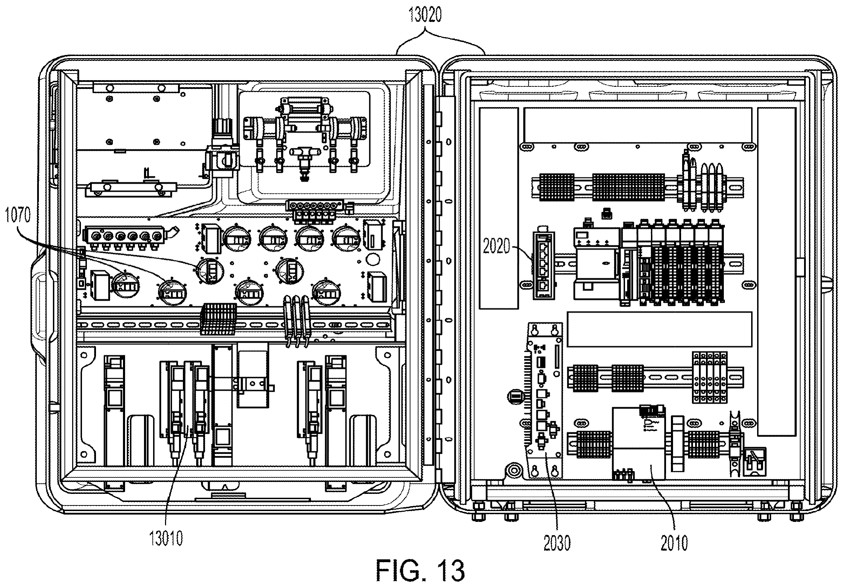

The system includes durable parts of the system receiving and supporting disposable single use components that are in direct fluid contact or part of the fluid pathway. Within the enclosure behind the primarily-disposable components are air cylinders to provide air to the biopsy vial, valves to control the fluid path, step-servo motors that drive actuators for the syringe pumps and the aspirator tube gripper assembly. The durable components further include the durable electronic components of the system as shown in FIG. 13 of the present teachings. Included are, for example, PLC 2010 contains a processor, power supplies 13010, Ethernet controller 2020, valves 1070 and input/output modules 2030. In an aspect within the durable enclosure 13020, controller 2020, which receives data from and sends commands to I/O module 2030, is electrically coupled to other parts of the system. Solid state relays electrically activate components of the system, NPN output connects the power supply to sensors, and DIN rail mount terminal block connects equipment to power wiring, are mounted upon a DIN rail that connects multiple components to a single datalogger terminal, forms a power bus, and/or enables equipment installation. In an aspect, within the durable enclosure 13020 is step-servo motor drive(s) coupled with a step-servo motor(s) to provide position and velocity control of actuator while tolerating wide fluctuations in load inertia and frictional loading. Also located within the durable enclosure are circuit breakers and power supplies providing power safely to components of the system.

In general, the flow of fluid from the source containers is to a syringe pump, then into the biopsy vial. The fluid is pumped by syringe pumps, and the path is set by open and closed valves. The system controller decides, based on the desired function of the system, which fluid to pump, which syringe is receiving the fluid, and therefore which actuator is controlling the syringe plunger, and which (in the case of multiple) biopsy vial into which the fluid is destined. In an aspect, other types of pumps can be used, for example, a peristaltic pump and/or a pneumatic pump, based on the desired fluid accuracy and cell viability percentage during the pumping process. In general, the flow of fluid from the biopsy vial includes paths from the vial to a syringe and then to the sample container (depositing transport fluid), from the vial to a syringe and then to the waste container, to/from the vial to a syringe during a biopsy recovery process, and from the vial to a syringe and then to the cell culture flask. Other paths are contemplated by the present teachings, as fluid flow in the system is completely general and flexible.

In an aspect, when the process involves moving the biopsy vial to the centrifuge (versus moving the contents of the biopsy vial to the centrifuge), the biopsy vial components of the present teachings accommodate such a move. The biopsy vial includes a centrifuge clip to maintain the aspiration tube in a desired location. The biopsy vial tubing is wrapped around the biopsy vial and sealed in place, and the bellows are sealed around the movable tube. The biopsy vial is closed throughout the centrifuge process, and the bellows limit the movement of the movable tube during centrifugation.

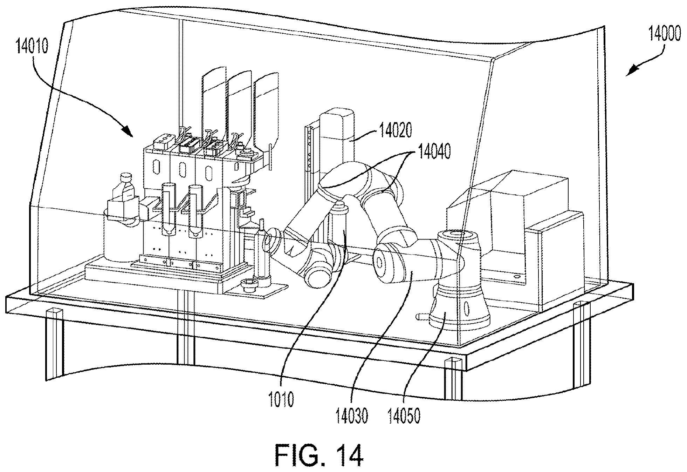

Referring now to FIG 14, an alternate embodiment of the system 14000 is shown to also include shown is a pump tower 14010 for the various fluid delivery operations discussed previously in which biopsy vial 1010 is positioned to be capped by capper 14020, to have the vial cap of the present teachings installed. The capped biopsy vial is installed in the closed pumping tower 14010 that performs fluid transfers as described herein. In an aspect, pump tower 14010 includes media containers, biopsy vial mounting stations, cell culture flask, all as previously described, biopsy vial capping station 14020, among other features.

A robotic arm 14030 is surrounded by various components performing the functions of the disclosure. Robotic arm 14030 loads biopsy vials 1010 into the desired system components. In an aspect, a single robotic arm 14030 can provide biopsy vial loading for several systems. In other configurations, a robotic arm 14030 can be dedicated to a single system, or a subgroup of systems. Robotic arm 14030 can grasp biopsy vial 1010 at pre-selected positions on biopsy vial, for example, at an upper cap gripper location. Robotic arm 14030 can include one or more articulated joints 14040 and rotatable base 14050 to enable servicing multiple systems. For example, if two systems are positioned side-by-side, robotic arm 14030 would only need a small rotation arc to accommodate both systems. At the same time, if removable components of system are located on opposite sides of system, the system can rotate either clockwise or counterclockwise until the component to be replaced is within the reach of robotic arm 14030. To accommodate the difference in vertical placement between, for example, cell culture flask and biopsy vial in the system, robotic arm 14030, in an aspect, includes a vertical reach range. In an aspect, biopsy systems can include a reduced planar footprint and elongated vertical footprint compared to other configurations described herein in order to accommodate side-by-side placement of multiple biopsy systems.

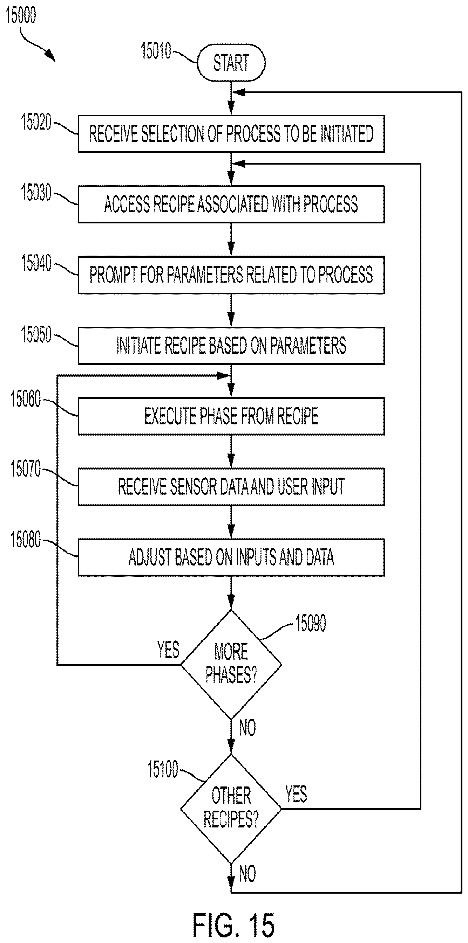

Referring now to FIG. 15, exemplary method 15000 for executing the phases of starting a project to be executed 15010 a project includes receiving 15020 a selection of the process to be initiated. In an aspect, the selection is provided by a human at a human machine interface. In an aspect, the selection is made dynamically by the system while completing a multi-process task. In an aspect, both human and system selections are made cooperatively. Method 15000 includes accessing 15030 a recipe associated with the process. In an aspect, the recipe is determined through a look-up table that associates the desired process with a recipe for carrying out the process. The recipe includes phase ordering, for example. Recipes can be dynamically formulated, depending upon the state of the system. Method includes accessing, receiving, or prompting for 15040 parameters related to the process. Parameters such as set points can be pre-determined, determined dynamically, or user-supplied. Method includes initiating 15050 the recipe based at least upon the parameters. The parameters are used to execute the recipe for the particular biopsy sample and desired environmental conditions. Method includes executing 15060 a phase from the recipe. The recipe includes steps or phases, each of which expects to receive information required to perform the phase. Method includes receiving 15070 sensor data and possibly user input associated with the phase. Method includes adjusting 15080 characteristics and parameters based at least upon the sensor data and user inputs. When a phase is complete. if 15090 there are more phases to process, method 15000 includes returning to step 15060 to process the next phase in the recipe. If 15090 there are no more phases to process, and if 15100 there are other recipes, method 15000 returns to step 15030 to access the next recipe to process. If 15100 there are no more recipes, method 15000 returns to step 15020 to receive another selection of a process to be initiated.

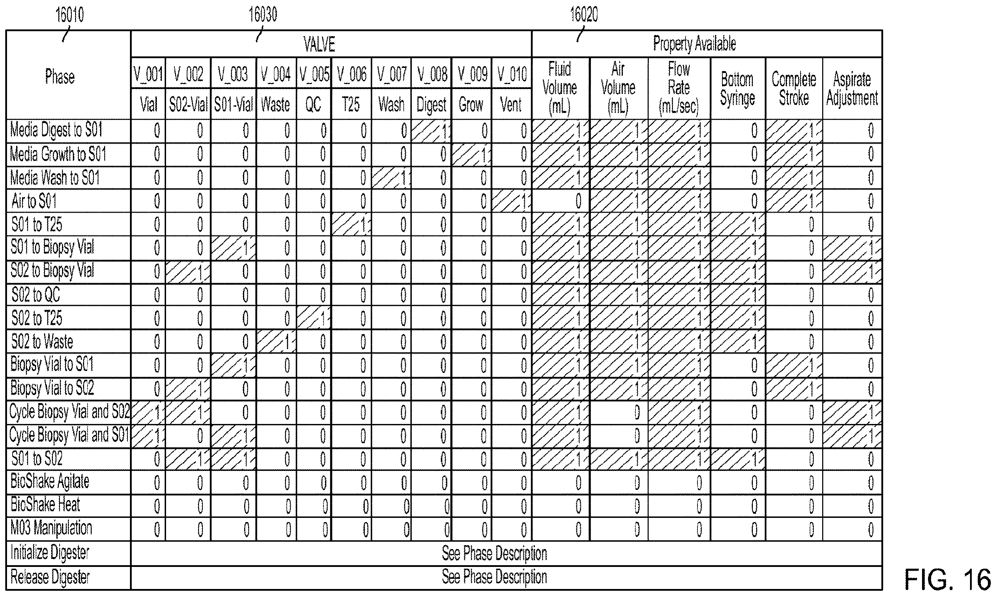

Referring now to FIG. 16, an exemplary list of phases 16010 for a particular recipe is shown. In the exemplary recipe, each of phases 16010 requires a set of instructions to be executed by PLC. PLC, for example, commands valves to open or close, resulting in properties 16020 being updated. The list of phases 16010 includes entries that refer to "S01" and "S02" which indicate input (S01) and output (S02) syringes as described herein. The biopsy vial in the list is the container in which the biopsy sample is held. Fluids are mixed in the biopsy vial, and the biopsy vial is placed in a centrifuge. For example, in a wash cycle, in which the biopsy is cleared of transport fluid, wash media is moved into S01, S01 delivers the wash media to the biopsy vial, and S02 cycles the fluid between S02 and the biopsy vial. To enable this fluid movement, the exemplary system includes three stepservo motor drives, M01, M02, and M03. M01 moves the S01 syringe, M02 moves the S02 syringe, and, in a phase, M03 moves (manipulates) the biopsy vial tube. In an aspect, the manipulation includes moving the tip of the biopsy vial tube, or modifying the grippers of the biopsy vial tube, as described herein. In an aspect, valves 16030 are associated with, for example, enabling tasks associated with moving fluid into/out of vials, and waste, sample (QC), culture (T25), wash, digestion, growth, and ventilation processes. In an aspect, properties 16010 include fluid volume, air volume, flow rate, and bottom syringe, completed stroke, and aspirate adjustment status. The table includes a binary value indicating whether or not valve 16030 is used and property 16020 is available in the listed phase 16010. In general, phases 16010 have similar sequences and vary in valve configuration and properties updated.

Continuing to refer to FIG. 16, in the exemplary recipe, properties associated with syringe command and movement include air volume, fluid volume, and flow rate. In the exemplary system, a bubble sensor and a measurement of the fluid flow rate are used to measure fluid and air volumes. PLC 205 receives sensor data from the bubble sensor which indicates if air or fluid are being transferred into or out of the biopsy vial. The volume of fluid is totalized using the bubble sensor data and the flow rate. In an example, if the desired value of either the air volume or the fluid volume is 0ml, but not both, PLC commands the syringe to move exactly as needed to deliver the nonzero volume. In another example, if the desired air volume is 0ml, and the desired fluid volume is, for example, 5ml, the syringe delivers 5ml of fluid. In another example, if the desired air volume is 2ml and the desired fluid volume is 5ml, the syringe delivers fluid until the air volume is 2ml. In other words, whichever goal is met first is when delivery ceases. Because there is only a single bubble sensor in the exemplary system, only one goal can be met at a time. In another example, if the desired air volume is 2ml and the fluid volume is 3.5ml, PLC commands the syringe to stop because the desired air volume has been met if the fluid volume is 3.5ml. It is possible that the measured air volume and/or fluid volume is irrelevant to the particular function. In that case, a preselected value can be used to indicate that no modification to either volume is necessary. In an aspect, when the pre-selected value is recognized, the fluid transfer is terminated when the fluid measures 5mL or the air volume causes the syringe to become fully extended. In an aspect, the user can configure an alarm if fluid or air volume is met or not.

Continuing to refer to FIG. 16, the flow rate property applies to both fluid and air flows in the system. In an aspect, the flow rate input range is, for example, 0.001-4 mL/sec and is valid for both fluid and air. The flow rate sets the speed of motor drives. The value of the bottom syringe property is used to drive syringe to the bottom, delivering the volume in syringe into the selected path. In an aspect, the bottom syringe property is not dependent upon fluid or air volume set points. In an aspect, the flow rate property is used to set the speed of syringe. The value of the complete stroke property is set by setting the air or fluid volume to a pre-selected value, for example 100. For example, to clear the syringe of either air or fluid, air volume is set to 100 and fluid volume is set to 0, or air volume is set to 0 and fluid volume is set to 100, or both air and fluid volumes are set to 100. The direction of the syringe is set to either push or pull fluid, and the result is that the syringe moves to one of its ends. Since the pre-selected value is chosen to be greater than the volume of the syringe, in the system of the present teachings, the syringe stops when the first non-zero volume is met. The aspirate adjustment property is used to make other properties available such as, for example, the state of gripper (no change, open, closed), moving gripper to adjust the height of tube (no change, top, where gripper grabs tube, bottom of biopsy vial, any height in distance, any height in the time it takes to reach the height), and the speed of tube.

Continuing to refer to FIG. 16, the agitate phase configures an agitation rate and duration. The heat phase configures a temperature set point, which involves applying heating or cooling to, for example, media, for a pre-selected amount of time. The motor manipulation phase enables commands to the digester that involve the aspirate assembly, such as, for example, but not limited to, setting a setpoint move position (e.g. a gripper target position in mm or counts), and a setpoint move time (e.g. the duration over which the move takes place).

Continuing to refer to FIG. 16, the digester initializing phase can be executed outside of a recipe, possibly a result of a user request, or within a recipe. In an aspect, steps to initialize the digester include, but are not limited to including, disabling motors, opening the gripper, opening all valves, prompting waits for the user to indicate the disposable is loaded, venting the disposable, enabling the motors, homing the motors, enabling the tube sensor, and moving the gripper downward by a pre-selected amount. If the tube is observed, moving the gripper to a pre-selected height and closing the gripper. If the tube is not observed, maintaining the gripper in an open position, and moving the gripper to a previous gripper grab height. If the tube is grabbed successfully or if the gripper is at the correct height to close the aspirate tip, and determining how much air and at what flowrate should the air be pushed into the vial through the ending of the aspirate tip. The initialization process includes closing the gripper and moving the aspirate tip within the biopsy vial.

Continuing to refer to FIG. 16, the digester release phase can be executed outside of a recipe, possibly as a result of a user request, or within a recipe. In an aspect, steps to release the digester include, but are not limited to, opening the gripper, disabling the motors, opening the valves, ensuring that the disposable is removed, and closing the valves.

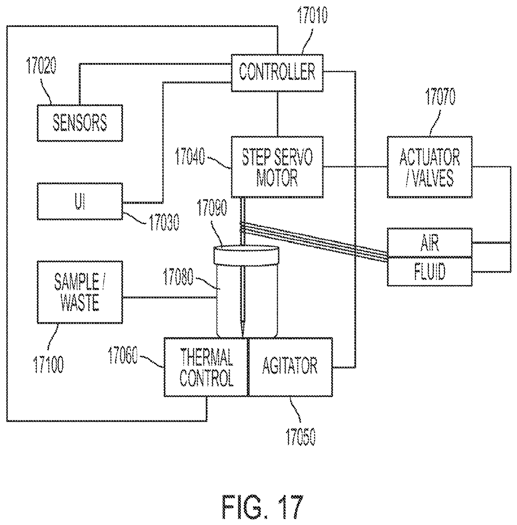

Referring now to FIG. 17, an exemplary schematic diagram of the components of the system of the present teachings and their interconnections are shown. Controller 17010 coordinates the activities of sensors 17020, user interface 17030, actuator motor 17040, agitator 17050, and media thermal control 17060. Specifically, controller 17010 receives sensor and user input that, with a recipe and state information available to controller 17010, indicate which commands to issue to, for example, motor(s) 17040. Motors 17040 control the state of actuators/valves 17070, as well as the mechanical and fluid interface with vial 17080 through cap 17090. Fluid can be provided, for example, to sample or waste containers 17100. Sensors 17020 provide information to controller 17010 that indicate when, for example, agitator 17050 should be activated as described herein. Sensors 17020 provide information to controller 17010 that indicate when thermal control 17060 should be activated to adjust fluid temperature in the system.

Various alternatives and modifications can be devised by those skilled in the art without departing from the disclosure. Accordingly, the present disclosure is intended to embrace all such alternatives, modifications and variances. Additionally, while several example configurations of the present disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular configurations. In addition, those skilled in the art will envision other modifications within the scope and spirit of the claims appended hereto. Other elements, steps, methods and techniques that are insubstantially different from those described above and/or in the appended claims are also intended to be within the scope of the disclosure.

The drawings are presented only to demonstrate certain examples of the disclosure. And, the drawings described are only illustrative and are non-limiting. In the drawings, for illustrative purposes, the size of some of the elements may be exaggerated and not drawn to a particular scale. Additionally, elements shown within the drawings that have the same numbers may be identical elements or may be similar elements, depending on the context.

Where the term "comprising" is used in the present description and claims, it does not exclude other elements or steps. Where an indefinite or definite article is used when referring to a singular noun, e.g. "a" "an" or "the", this includes a plural of that noun unless something otherwise is specifically stated. Hence, the tenn "comprising" should not be interpreted as being restricted to the items listed thereafter; it does not exclude other elements or steps, and so the scope of the expression "a device comprising items A and B" should not be limited to devices consisting only of components A and B.

Furthermore, the terms "first", "second", "third," and the like, whether used in the description or in the claims, are provided for distinguishing between similar elements and not necessarily for describing a sequential or chronological order. It is to be understood that the terms so used are interchangeable under appropriate circumstances (unless clearly disclosed otherwise) and that the example configurations of the disclosure described herein are capable of operation in other sequences and/or arrangements than are described or illustrated herein.
The present application is a divisional application originating from EP24717998.9.
EP24717998.9 originates from PCT/US2024/021063. The original claims of PCT/US2024/021063 are presented as statements below so that the subject-matter of those 49 claims is included in its entirety in the present application.
Statement 1. A system for automated digestion of a biopsy comprising:
   a durable subsystem configured to control a flow of fluids and air to the biopsy; and a disposable subsystem configured to provide the fluids and air to the biopsy, the fluids washing and digesting the biopsy, the washing an
   d digesting forming digested cells.
Statement 2. The method as in claim 1 wherein the disposable subsystem comprises:
   a biopsy vial holding the biopsy;
   a biopsy vial cap configured to accept the fluids and air through a vial tube, the vial tube configured to move within the biopsy vial; and
   at least one syringe pump moving the fluids to and from the biopsy vial through the vial tube, the fluids and the air moving the biopsy within the vial tube.
Statement 3. The method as in statement 2 wherein the durable subsystem comprises:
   at least one actuator configured to move the vial tube within the biopsy vial;
   an agitator configured to provide agitation to the biopsy vial, the agitation separating cells of the biopsy from each other; and
   a controller configured to command movement of the fluids to and from the biopsy vial.
Statement 4. The method as in statement 3 wherein the fluids comprise:
   digestion fluids, the digestion fluids creating digested cells from the separated cells.
Statement 5. The method as in statement 4 wherein the fluids comprise:
   wash fluids, the wash fluids clearing a transport fluid from the biopsy, the wash fluids preparing the biopsy for the digestion fluids.
Statement 6. The method as in statement 1 wherein the disposable subsystem comprises:
   at least one syringe configured to accept the fluids.
Statement 7. The method as in statement 1 wherein the disposable subsystem comprises:
   a cell culture flask configured to receive combined digested cells after centrifugation.
Statement 8. The method as in statement 3 wherein the disposable subsystem comprises:
   a waste container configured to receive the fluids from the biopsy vial when the controller determines the fluid is to be discarded.
Statement 9. The method as in statement 3 wherein the disposable subsystem comprises:
   a sample container configured to receive the fluids from the biopsy vial when thecontroller determines the fluids are to be tested.
Statement 10. The method as in statement 3 further comprising:
   at least one step-servo motor controlled by the controller, the at least one step-servo motor controlling a volume and a flow rate of the fluid.
Statement 11. The method as in statement 10 wherein the at least one step-servo motor comprises:
   controlling a height of the vial tube within the biopsy vial.
Statement 12. The method as in statement 3 further comprising:
   at least one valve controlled by the controller, the at least one valve controlling a flow path of the fluid.
Statement 13. A method for automatically digesting cells comprising:
   receiving a biopsy including the cells and a transport fluid in a biopsy vial;
   attaching a cap to the biopsy vial, the cap including a vial tube, the cap forming an interior of the biopsy vial and an exterior of the biopsy vial, a position of the vial tube being adjustable, the vial tube extending from the interior to the exterior, the interior being sealed from the exterior;
   pumping air into the biopsy vial through the vial tube, the air moving the biopsy within the biopsy vial;
   pumping the transport fluid to a container;
   pumping wash fluid into the biopsy vial through the vial tube;agitating the biopsy vial;
   pumping the wash fluid to the container;
   pumping a digester fluid into the biopsy vial through the vial tube; and
   centrifuging the digester fluid and the agitated washed cells forming digested cells.
Statement 14. The method as in statement 13 further comprising:
   seeding a cell culture flask with the digested cells.
Statement 15. The method as in statement 13 further comprising:
   moving, by an actuator and grippers, the vial tube within the biopsy vial, the cap including a gripper feature, the gripper feature configured to enable the grippers to grab and release the gripper feature and move the vial tube out of and into the biopsy vial.
Statement 16. The method as in statement 13 wherein the cap comprises:
   vents configured to release and admit air from/to the biopsy vial.
Statement 17. The method as in statement 16 the cap comprises:
   at least one filter configured to filter the air.
Statement 18. The method as in statement 13 wherein the cells comprise:
   tissue from a patient.
Statement 19. The method as in statement 14 further comprising:
   incubating the seeded cells.
Statement 20. The method as in statement 19 further comprising:
   providing the incubated cells to a patient.
Statement 21. The method as in statement 13 further comprising:
   determining a position of the vial tube based at least on a sensor.
Statement 22. A system for automatically processing cell samples, comprising:
   a vial containing a biopsy sample positioned within said system;
   a cap on said vial, said cap having a moveable tube extending through an opening therein;
Statement 23. A system for digesting a biopsy sample, comprising:
   a vial configured to receive the biopsy sample and a transport fluid;
   a cap coupled to the vial, the cap comprising a movable tube having an aspiration tip at a distal end;
   a tube gripper configured to engage a neck of the movable tube and move the movabletube up and down within the vial;
   an input syringe pump configured to pump air into the vial through the movable tube andlo supply a wash fluid and a digest solution to the vial through the movable tube;
   an output syringe configured to withdraw the transport fluid, the wash fluid, and thedigest solution from the vial through the movable tube;
   a fluid reservoir operably coupled with the input syringe pump;
   a sensor configured to detect solids in the fluid withdrawn by the output syringe; and
   a controller configured to control the movement of the tube gripper, the input syringe pump, and the output syringe.
Statement 24. The method of statement 23, wherein the controller executes a recovery procedure when thesensor detects solids in the fluid withdrawn by the output syringe, the recovery procedure comprising providing additional fluid lo the vial from the fluid reservoir to return the solids tothe vial.
Statement 25. The method of statement 23, wherein the vial is configured to receive a biopsy sample from a digestive organ.
Statement 26. The method of statement 23, wherein the cap further comprises a valve that controls the flow of fluid through the movable tube.
Statement 27. The system of statement 23, wherein the controller is further configured to agitate the vial by moving the tube gripper in a circular motion.
Statement 28. The method of statement 23, wherein the controller is further configured to centrifuge the vial to separate the cells from the digest solution.
Statement 29. The method of statement 23, wherein the sensor is an optical sensor that detects the presence of solids by measuring the light transmittance of the fluid.
Statement 30. A method for executing a process on a biopsy sample, comprising:
   receiving a selection of the process to be initiated, wherein the selection is provided bya human at a human machine interface, or made dynamically by a system while completing a multi-process task, or both;
   accessing a recipe associated with the process, wherein the recipe is determined through a look-up table that associates the desired process with a recipe for carrying out the process, or dynamically formulated depending on the state of the system, or both, and whereinthe recipe includes phase ordering;
   accessing, receiving, or prompting for parameters related to the process, wherein the parameters include set points that are pre-determined, determined dynamically, or user-supplied, or any combination thereof;
   initiating the recipe based at least upon the parameters, wherein the parameters are usedto execute the recipe for the particular biopsy sample and desired environmental conditions;
   executing a phase from the recipe, wherein the recipe includes steps or phases, each of which expects to receive information required to perform the phase;
   receiving sensor data associated with the phase;
   adjusting characteristics and parameters based at least upon the sensor data;
   repeating the executing, receiving, and adjusting steps for each subsequent phase in the recipe until all phases are completed; and
   if there are other recipes to be executed, returning to the accessing step to access the next recipe to process, or if there are no more recipes, returning to the receiving step to receiveanother selection of a process to be initiated.
Statement 31. The method of statement 30, wherein the process is selected from the group consisting of: wash, digestion, growth, and ventilation.
Statement 32. The method of statement 30. wherein the recipe is accessed from a database stored in a memory of the system.
Statement 33. The method of statement 30, wherein the parameters include at least one of: fluid volume,air volume, flow rate, and bottom syringe.
Statement 34. The method of statement 30, wherein the sensor data includes at least one of: temperature,pressure, pH, and optical density.
Statement 35. The method of statement 30, wherein the adjusting step comprises controlling at least one of: a valve, a syringe, a motor, and a centrifuge.
Statement 36. The method of statement 30, wherein the biopsy sample is held in a biopsy vial that is manipulated by the system.
Statement 37. The method of any one of statement 30, wherein the human machine interface comprises atouch screen, a keyboard, a mouse, a microphone, a speaker, or a combination thereof.
Statement 38. A system for processing a biopsy containing sells, comprising:
   a biopsy vial containing a biopsy tissue and a transp011 fluid and having a transport cap;
   a replacement cap to replace said transport cap, said replacement cap having a movable tube extending therethrough;
   a fluid supply subsystem; and
   a tubing cassette fluidically connecting said fluid supply subsystem to said movable tube.
Statement 39. The method of statement 38. further comprising:
   a user interface for adjusting operating parameters, controlling the process andproviding reporting on the process.
Statement 40. The method of statement 38, wherein said biopsy vial, said replacement cap. said fluid supply subsystem and said tubing cassette are connected to form a closed sterile loop,
Statement 41. The method of statement 40, further comprising:
   at least one pump to move fluid within said closed loop.
Statement 42. The method of statement 41, wherein said at least one pump is a syringe pump.
Statement 43. The method of statement 41, wherein said at least one pump is two syringe pumps.
Statement 44. The method of statement 38, further comprising:a biopsy vial subsystem including:
   said biopsy vial;
   said replacement cap with said movable tube therein;
   a gripper engaged with said movable tube;
   an actuator for movement of said gripper;
   sensors; and
   an agitator.
Statement 45. The method of statement 44, wherein the sensors are laser sensors for monitoring the position of biopsy vial movable tube and to detect the presence of solids among the liquid passing through biopsy vial movable tube,
Statement 46. 'The method of statement 45, wherein a solid recovery operation is executed ·when a solid is detected.
Statement 47. The method of statement 38, the fluid supply subsystem further comprising:
   a fluid bag mount supporting a plurality of fluid bags.
Statement 48. The method of statement 47, the fluid supply subsystem further comprising:
   thermal control elements to maintain said fluid bags at a predetermined temperature.
Statement 49. The method of statement 38, the tubing cassette further comprising:
   a cassette body having a lubing path therein;
   a dosed tubing loop within said tubing path;
   openings within said cassette body that position said closed tubing loop such that it isover and accessible to a plurality of pinch valves.

## Claims

1. A system for digesting a biopsy sample, comprising:
a vial configured to receive the biopsy sample and a transport fluid;
a cap coupled to the vial, the cap comprising a movable tube having an aspiration tip at a distal end;
a tube gripper configured to engage a neck of the movable tube and move the movable tube up and down within the vial;
an input syringe pump configured to pump air into the vial through the movable tube and to supply a wash fluid and a digest solution to the vial through the movable tube;
an output syringe configured to withdraw the transport fluid, the wash fluid, and the digest solution from the vial through the movable tube;
a fluid reservoir operably coupled with the input syringe pump;
a sensor configured to detect solids in the fluid withdrawn by the output syringe; and
a controller configured to control the movement of the tube gripper, the input syringe pump, and the output syringe.

2. The system of Claim 1, wherein the controller is configured to execute a recovery procedure when the sensor detects solids in the fluid withdrawn by the output syringe, the recovery procedure comprising providing additional fluid to the vial from the fluid reservoir to return the solids to the vial.

3. The system of Claim 1, wherein the vial is configured to receive a biopsy sample from a digestive organ.

4. The system of any of the preceding claims, wherein the cap further comprises a valve that controls the flow of fluid through the movable tube.

5. The system of any of the preceding claims, wherein the controller is further configured to agitate the vial by moving the tube gripper in a circular motion.

6. The system of any of the preceding claims, wherein the controller is further configured to centrifuge the vial to separate the cells from the digest solution.

7. The system of any of the preceding claims, wherein the sensor is an optical sensor that detects the presence of solids by measuring the light transmittance of the fluid.
